# EUROPEAN PATENT APPLICATION

(11) **EP 4 696 722 A1**
(43) Date of publication of application: **18.02.2026**
(21) Application number: 24788764.9
(22) Date of filing: 10.04.2024
(51) Int. Cl.: C08F 20/28, B32B 27/30, C08F 290/06, C08G 64/02, C09K 9/02, G02B 5/23, G02C 7/02, G02C 7/10

(54) **(METH)ACRYLATE, CURABLE COMPOSITION, CURED BODY, LAMINATE, OPTICAL ARTICLE, LENS, AND EYEGLASSES**

(30) Priority: 12.04.2023 JP 2023065261
(71) Applicant: TOKUYAMA CORPORATION, Yamaguchi 745-8648 (JP)
(72) Inventor: MIYAZAKI, Masayuki, Shunan-shi, Yamaguchi 745-8648 (JP); HIRAREN, Toshimitsu, Shunan-shi, Yamaguchi 745-8648 (JP)
(74) Representative: Kraus & Lederer PartGmbB
(86) International application number: PCT/JP2024/014533
(87) International publication number: WO 2024/214740

(57) **Abstract**

The purpose of the present disclosure is to provide a (meth)acrylate capable of achieving the excellent performance of functional dyes, a curable composition, a cured product, a laminate, an optical component, a lens, and eyeglasses. According to an embodiment, a (meth)acrylate represented by Formula (1A) is provided. According to another embodiment, a curable composition is provided. The curable composition contains a (meth)acrylate according to the one embodiment and a functional dye. According to another embodiment, a cured product is provided. The cured product is obtained by curing the curable composition according to the one embodiment.

## Description

### TECHNICAL FIELD

The present invention relates to a (meth)acrylate, a curable composition, a cured product, a laminate, an optical component, a lens, and eyeglasses.

### BACKGROUND ART

Photochromic compounds, such as naphthopyran compounds, fulgid compounds, and spirooxazine compounds, have two isomers with different absorption spectra, one of which will reversibly turn into the other upon exposure to UV-containing light, such as sunlight or mercury lamp light. In general, photochromic compounds are characterized in that, upon exposure to UV light, they will undergo isomerization and change their color from colorless to colored (photochromic reaction) and that, when placed in a dark place after being exposed to light, they will recover their original color (hereinafter such properties are also referred to as photochromic properties). Such properties enable them to find various applications, especially optical material applications.

For example, photochromic compounds are used to form photochromic eyeglass lenses with photochromic properties. Outdoors, when exposed to UV-containing light, such as sunlight, such lenses will rapidly become colored and function as sunglass lenses, and indoors, without such exposure to light, they will become colorless and function as normal eyeglass lenses. The demand for such lenses has been increasing in recent years.

Photochromic eyeglass lenses are produced, for example, by a process including: applying a photochromic curable composition to plastic lenses by spin coating or other techniques; and photo-curing the composition to form a photochromic coating layer.

### Citation List

### Patent Document

Patent Document 1: PCT International Publication No. WO98/37115
Patent Document 2: US Patent No. 5914174
Patent Document 3: PCT International Publication No. WO01/02449
Patent Document 4: PCT International Publication No. WO03/11967
Patent Document 5: PCT International Publication No. WO2015/054036
Patent Document 6: PCT International Publication No. WO2009/075388
Patent Document 7: Japanese Unexamined Patent Application, Publication No. 2020-172565
Patent Document 8: Japanese Unexamined Patent Application, Publication No. 2022-120570
Patent Document 9: PCT International Publication No. WO2014/136804

### DISCLOSURE OF THE INVENTION

### Problems to be Solved by the Invention

It is an object of the present invention to provide a (meth)acrylate, a curable composition, a cured product, a laminate, an optical component, a lens, and eyeglasses, each of which allows a functional colorant to exhibit high performance.

### Means for Solving the Problems

The present disclosure provides a (meth)acrylate represented by Formula (1A) below.

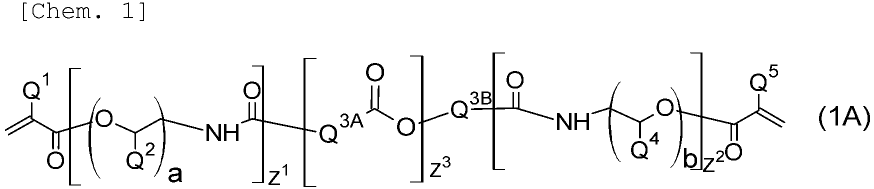

In Formula (1A), Q¹ and Q⁵ are each independently a hydrogen atom or a methyl group,
Q² and Q⁴ are each independently a hydrogen atom or an alkyl group having 1 to 3 carbon atoms,
Q^{3A} and Q^{3B} are each independently a divalent group represented by Formula (1a) below,
when two or more Q^{3A} are present, the two or more Q^{3A} may be the same group or different groups,
a and b are each independently 0 or more and 10 or less, and Z¹ and Z² are each independently 0 or 1, and
Z³ is 1 to 100.

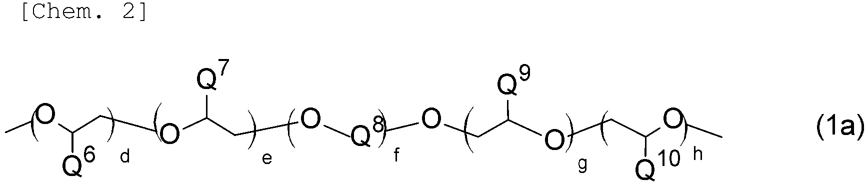

In Formula (1a), Q⁶ and Q¹⁰ are each a hydrogen atom or an alkyl group having 1 to 3 carbon atoms,
Q⁷ and Q⁹ are each a hydrogen atom or an alkyl group having 1 to 3 carbon atoms,
Q⁶ and Q⁷ are different groups,
Q⁹ and Q¹⁰ are different groups,
Q⁸ is an optionally substituted, linear or branched alkylene group having 2 to 20 carbon atoms,
d and h are each 0 or more and 10 or less,
e and g are each 0 or more and 20 or less, and
f is 3 or more and 100 or less.

The present disclosure provides a curable composition. The curable composition includes the (meth)acrylate according to an embodiment and a functional colorant.

The present disclosure provides a cured product. The cured product includes a product resulting from curing of the curable composition according to an embodiment.

The present disclosure provides a laminate. The laminate includes an optical base material and the cured product according to an embodiment provided on a surface of the optical base material.

The present disclosure provides an optical component. The optical component includes the cured product according to an embodiment.

The present disclosure provides a lens. The lens includes the cured product according to an embodiment.

The present disclosure provides eyeglasses. The eyeglasses include the lens according to an embodiment.

### Effects of the Invention

The present invention provides a (meth)acrylate, a curable composition, a cured product, a laminate, an optical component, a lens, and eyeglasses, each of which allows a functional colorant to exhibit high performance.

### BRIEF DESCRIPTION OF THE DRAWINGS

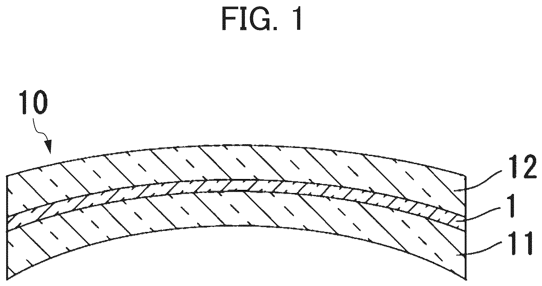

FIG. 1 is a schematic cross-sectional view of an example of the laminate according to an embodiment.

### PREFERRED MODE FOR CARRYING OUT THE INVENTION

An embodiment provides a (meth)acrylate represented by Formula (1A). The (meth)acrylate is suitable for use as a material for forming a resin composition containing a functional colorant. It will be described why the (meth)acrylate is suitable for use as such a composition.

First of all, the functional colorant includes a compound that has the ability to selectively absorb visible rays and develops color, becomes colorless, or changes color upon exposure to light, heat, electric field, pressure, or any other type of energy. The functional colorant with such features changes its structure under specific conditions to provide a specific function. In general, a cured plastic material has a rigid matrix structure. As compared with a solution, such a cured material has only a small free space for the functional colorant to change its structure. In such a cured material, therefore, the functional colorant can less easily change its structure than in a solution and can function in a limited manner.

The (meth)acrylate represented by Formula (1A) is characterized by having a structure in which the Q^{3A} and Q^{3B} moieties are bonded via a carbonate bond. The Q^{3A} and Q^{3B} moieties each have a repeating structure represented by -(O-Q⁸)_{f}-, which is referred to as a first alkylene oxide chain having 2 or more and 20 or less carbon atoms. In a cured product, the first alkylene oxide chain can act as a soft segment to form a free space for the functional colorant. Thus, when placed near the soft segment of the first alkylene oxide chain, the functional colorant will be less likely to be hindered from changing its structure and thus will exhibit high performance. The (meth)acrylate according to an embodiment has a carbonate bond between the Q^{3A} and Q^{3B} moieties, which each have the first alkylene oxide chain capable of acting as a soft segment. Thanks to a strong cohesive force between the carbonate bonds, the soft segments can easily form an aggregate structure. The (meth)acrylate according to an embodiment can also form a cured product that not only allows the functional colorant to exhibit high functionality but also has high hardness. In this regard, a high-hardness cured product may have a low soft-segment content and a narrow free space, which may tend to reduce the functionality of the functional colorant. Even when the (meth)acrylate according to an embodiment forms a cured product with a low soft-segment content, however, the aggregate structure of the soft segments, which the first alkylene oxide chains can easily form, will provide a wide free space, which can prevent a reduction in the functionality of the functional colorant. Therefore, the (meth)acrylate according to an embodiment will form a cured product that not only allows the functional colorant to exhibit high performance but also has high hardness.

### (Meth)acrylate Represented by Formula (1A)

The (meth)acrylate according to an embodiment is represented by Formula (1A) below. The (meth)acrylate may be a diacrylate having two acryloyl groups, a dimethacrylate having two methacryloyl groups, or an acrylate-methacrylate having one acryloyl group and one methacryloyl group. The (meth)acrylate has at least one carbonate bond per molecule.

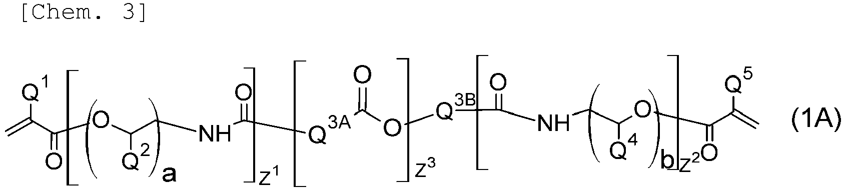

In Formula (1A), Q¹ and Q⁵ are each independently a hydrogen atom or a methyl group and are each preferably a methyl group.

In Formula (1A), Z³ is 1 or more and 100 or less. For good balance between functionality and hardness and for handling-friendly viscosity, Z³ is preferably 1 or more and 85 or less, more preferably 1 or more and 70 or less, even more preferably 1 or more and 50 or less, furthermore particularly 2 or more and 25 or less.

Q^{3A} and Q^{3B} are each independently a divalent group represented by Formula (1a) below. When two or more Q^{3A} are present, the two or more Q^{3A} may be the same group or different groups.

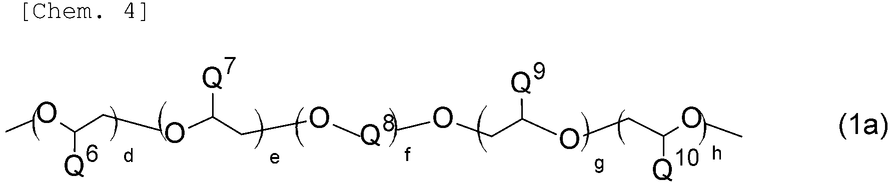

In Formula (1a), Q⁸ is an optionally substituted, linear or branched alkylene group having 2 or more and 20 or less carbon atoms. The alkylene group preferably has two or more carbon atoms, more preferably three or more carbon atoms, even more preferably four or more carbon atoms. The alkylene group may have 15 or less carbon atoms, 10 or less carbon atoms, or 6 or less carbon atoms. Q⁸ is preferably an unsubstituted linear alkylene group. The repeating unit -(OQ⁸)- with the subscript f represents a first alkylene oxide unit having 2 or more and 20 or less carbon atoms. The polymer moiety including the repeating units can form a soft segment in a cured product. The (meth)acrylate with the alkylene group having a larger number of carbon atoms can form a cured product with higher functionality. The (meth)acrylate with the alkylene group having too large a number of carbon atoms may provide a reduced amount of soft segments per unit mass and thus may form a cured product with reduced functionality.

In Formula (1a), f is 3 or more and 100 or less. For good balance between functionality and hardness, f is preferably 3 or more and 85 or less, more preferably 3 or more and 70 or less, even more preferably 3 or more and 50 or less, furthermore preferably 3 or more and 25 or less.

Q⁷ and Q⁹ are each a hydrogen atom or an alkyl group having 1 to 3 carbon atoms. Q⁷ and Q⁹ are each preferably a hydrogen atom or a methyl group. In Formula (1a), e and g are each 0 or more and 20 or less. In Formula (1a), e and g may each be 1 or more and 15 or less or 5 or more and 10 or less.

Q⁶ and Q¹⁰ are each a hydrogen atom or an alkyl group having 1 to 3 carbon atoms. Q⁶ and Q¹⁰ are each preferably a hydrogen atom or a methyl group. In Formula (1a), d and h are each 0 or more and 10 or less. In Formula (1a), d and h may each be 1 or more and 5 or less. In Formula (1a), d and h are 0 when e and g are 0.

Q⁶ and Q⁷ are different groups. Q⁹ and Q¹⁰ are different groups.

In other words, the (meth)acrylate according to an embodiment may further have at least one selected from the group consisting of a second alkylene oxide unit, which is the repeating unit with the subscript e or g, and a third alkylene oxide unit, which is the repeating unit with the subscript d or h. The urethane (meth)acrylate according to an embodiment may have a di- or tri-block structure having at least one selected from the group consisting of the second alkylene oxide unit and the third alkylene oxide unit. The urethane (meth)acrylate having such a di- or tri-block structure can form a cured product with a microphase-separated structure.

Z¹ and Z² are each independently 0 or 1. When at least one of Z¹ or Z² is 1, the (meth)acrylate represented by Formula (1A) is a urethane (meth)acrylate having at least one urethane bond.

Q² and Q⁴ are each independently a hydrogen atom or an alkyl group having 1 to 3 carbon atoms, and are each preferably a hydrogen atom.

In Formula (1A), a and b are each independently 0 or more and 10 or less. For example, a and b are each typically 1 or more and 5 or less and preferably 1 or 2.

To have handling-friendly viscosity, the (meth)acrylate according to an embodiment is preferably represented by Formula (1A) with Z¹ and Z² being 0. The (meth)acrylate with such a feature is represented, for example, by Formula (1B) below.

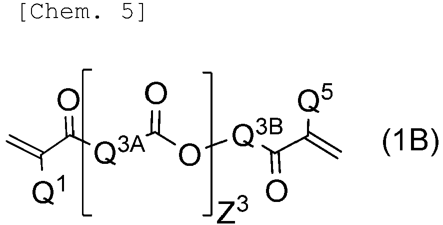

In Formula (1B), Q¹, Q^{3A}, Q^{3B}, Q⁵, and Z³ are as defined for Formula (1A) above.

To allow the functional colorant to exhibit higher performance, the (meth)acrylate according to an embodiment is preferably represented by Formula (1C) below.

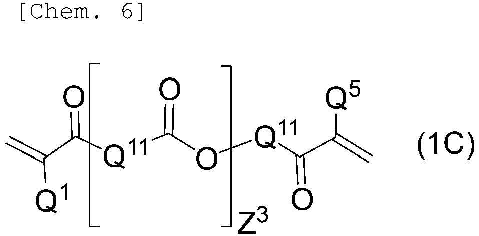

In Formula (1C), Q¹, Q⁵, and Z³ are as defined for Formula (1A) .

Q¹¹ is a divalent group represented by Formula (1b) below, and two or more Q¹¹ may be the same group or different groups.

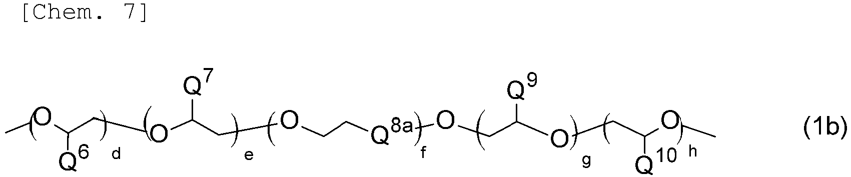

Q^{8a} is an optionally substituted, linear or branched alkylene group having 1 to 7 carbon atoms. The alkylene group preferably has 1 or more and 5 or less carbon atoms, more preferably 2 or more and 4 or less carbon atoms.

Q⁶, Q⁷, Q⁹, Q¹⁰, d, e, f, g, and h are as defined for Formula (1a).

The (meth)acrylate according to an embodiment preferably has only the first alkylene oxide unit. In other words, d, e, g, and h are preferably 0. The (meth)acrylate with such a feature will tend to form a cured product that not only allows the functional colorant to exhibit higher performance but also has desired hardness.

The (meth)acrylate with such a feature is represented, for example, by Formula (1D) below.

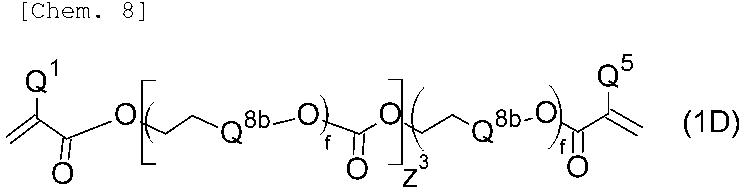

In Formula (1D), Q¹, Q⁵, and Z³ are as defined for Formula (1A), and f is as defined for Formula (1a).

Q^{8b} is a linear alkylene group having 1 to 7 carbon atoms. Q^{8b} is preferably a linear alkylene group having 1 to 5 carbon atoms, more preferably a linear alkylene group having 1 to 3 carbon atoms, most preferably a linear alkylene group having 2 carbon atoms.

Two or more - (CH₂CH₂Q^{8b}O)_{f}- groups may be the same or different. For ease of synthesis, two or more -(CH₂CH₂Q^{8b}O)_{f}-groups are preferably the same.

To form a cured product with desired hardness, the (meth)acrylate according to an embodiment preferably has a number-average molecular weight of 100 or more, more preferably 200 or more, even more preferably 400 or more. To allow the functional colorant to exhibit high functionality and to form a curable composition with handling-friendly viscosity, the (meth)acrylate preferably has a number-average molecular weight of 10,000 or less, more preferably 8,000 or less, even more preferably 6,000 or less, furthermore particularly 4,500 or less. The number-average molecular weight can be calculated, for example, using nuclear magnetic resonance (NMR) spectroscopy.

The ratio of the number-average molecular weight of the first alkylene oxide chain to that of the (meth)acrylate is preferably 20 mass% or more, more preferably 40 mass% or more, even more preferably 60 mass% or more. The (meth)acrylate with a higher first alkylene oxide chain molecular weight ratio will tend to form a cured product that allows the functional colorant to exhibit higher functionality. The ratio can be calculated, for example, using nuclear magnetic resonance (NMR) spectroscopy.

The (meth)acrylate according to an embodiment may be produced, for example, by the process described below. The process will be described for the production of the (meth)acrylate represented by Formula (1A).

First, a polyol compound represented by Formula (1c) below is provided. The polyol compound may be a plant- or petroleum-derived product. In Formula (1c), Q^{3A}, Q^{3B}, and Z³ are as defined for Formula (1A) above.

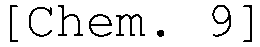

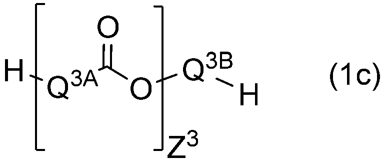

Next, a compound represented by Formula (1d) below is provided. The compound represented by Formula (1d) has one isocyanate group and one (meth)acryloyl group.

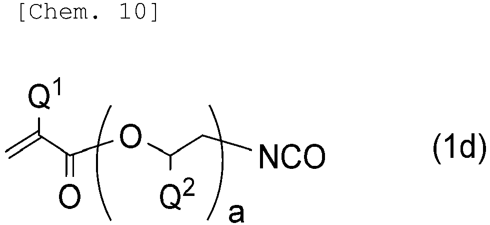

In Formula (1d), Q¹, Q², and a are as defined for Formula (1A).

The polyol compound represented by Formula (1c) is reacted with the isocyanate compound represented by Formula (1d) to form the (meth)acrylate represented by Formula (1A).

The reaction may be performed in the presence of a solvent. The solvent may be, for example, acetone, methyl ethyl ketone, methyl isobutyl ketone, diethyl ketone, cyclohexanone, dioxane, toluene, hexane, heptane, ethyl acetate, butyl acetate, dimethylformamide, or tetrahydrofuran.

Alternatively, the (meth)acrylate may be synthesized by esterification of (meth)acrylic acid. Specifically, the (meth)acrylate may be produced by a synthesis process including: reacting (meth)acrylic acid and the above polyol compound dissolved in a solvent, such as toluene, in the presence of a mineral acid, such as sulfuric acid or hydrochloric acid, an organic acid, such as aromatic sulfonic acid, or a Lewis acid, such as boron fluoride ether, while heating and stirring as needed and removing the produced water by azeotropy. In the esterification reaction, the method for removing water may include removing water with a drying agent, such as anhydrous magnesium sulfate or molecular sieves, or removing water in the presence of a dehydrating agent, such as dicyclohexylcarbodiimide.

Alternatively, the (meth)acrylate may be synthesized by esterification reaction with a (meth)acrylic acid halide. Specifically, the esterification reaction may include reacting acrylic acid and the polyol compound dissolved in an ether solvent, such as tetrahydrofuran, in the presence of a base, such as pyridine or dimethylaniline, while heating and stirring as needed; and removing the produced hydrogen halide.

Alternatively, the (meth)acrylate may be synthesized by ester exchange reaction with an ester compound, such as (meth)acrylic anhydride or methyl (meth)acrylate. Specifically, the ester exchange reaction may include reacting acrylic acid and the polyol compound dissolved in a solvent, such as toluene, in the presence of an acidic catalyst, such as aromatic sulfonic acid, or a basic catalyst, such as sodium acetate or pyridine, while heating and stirring as needed.

The polyol compound may be synthesized using the method described in Patent Document 7 or Patent Document 8.

Specifically, a polyol compound represented by H-Q^{3A}-H and a carbonate diester, such as alkylene carbonate, dialkyl carbonate, diaryl carbonate, or cyclic carbonate, may be subjected to easter exchange reaction at 70 to 250°C, preferably at 80 to 220°C, in the presence of an ester exchange catalyst, such as an acetic acid salt, nitric acid salt, sulfuric acid salt, carbonic acid salt, phosphoric acid salt, hydroxide, halide, acetylacetonate salt, or alkoxide of at least one metal selected from Group 2 of the periodic table.

For example, the polyol compound represented by H-Q^{3A}-H having a structure represented by Formula (1f) below may be synthesized by the method described below.

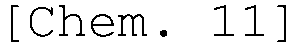

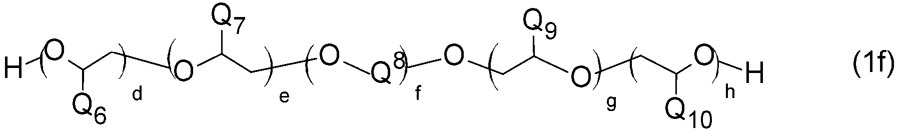

The polyol compound represented by Formula (1f) with d and h being 0 and e and g being 1 or more (namely the polyol compound having the second alkylene oxide unit) may be synthesized by reacting H-(OQ⁸)_{f}-OH with a cyclic ether compound, such as ethylene oxide or propylene oxide. For example, the polyol compound having the second alkylene oxide unit may be synthesized by performing the reaction at high temperature and high pressure in the presence of a catalyst, such as potassium hydroxide or any other alkali metal hydroxide, in an autoclave purged with nitrogen.

The polyol compound represented by Formula (1f) with d, e, g, and h being 1 or more (namely the polyol compound having the second and third alkylene oxide units) may be synthesized by the reaction of the cyclic ether compound with the polyol compound having the second alkylene oxide unit.

### Curable Composition

An embodiment provides a curable composition. The curable composition includes the (meth)acrylate according to an embodiment and a functional colorant.

The curable composition according to an embodiment may be used as a coating agent, an adhesive, a paint, or a material for plastic products, such as 3D printed products. In particular, the curable composition is suitable for use in optical component applications.

The curable composition preferably further includes a radically polymerizable monomer or monomers. The radically polymerizable monomer may be a first, second, third, fourth, or fifth radically polymerizable monomer described below.

Hereinafter, each of the components will be described in detail. Radically Polymerizable Monomer(s) (A) The radically polymerizable monomer(s) (A) may include a first radically polymerizable monomer represented by Formula (I) (a component (A-1)). Hereinafter, the first radically polymerizable monomer represented by Formula (I) will also be referred to as the component (A-1). The radically polymerizable monomer(s) (A) will also be referred to as the component (A). The radically polymerizable monomer(s) (A) may include any other radically polymerizable monomer depending on the desired properties of the cured product. The radically polymerizable monomer(s) (A) may include any known monomer polymerizable with the component (A-1). Such a monomer polymerizable with the component (A-1) is preferably a (meth)acrylate group-containing, radically polymerizable monomer, such as a radically polymerizable monomer having three or more (meth)acryloyl groups per molecule (component (A-2)) or any other (meth)acryloyl group-containing, radically polymerizable monomer (component (A-3)).

### Component (A-1): First Radically Polymerizable Monomer

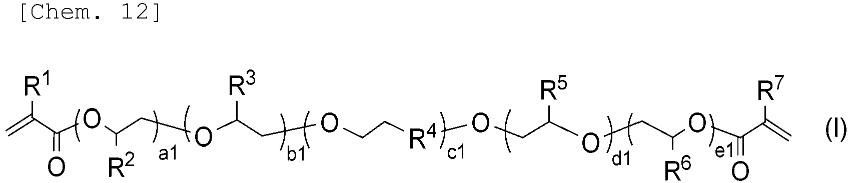

In Formula (I), R¹ and R⁷ are each independently a hydrogen atom or a methyl group. Specifically, the component (A-1) may be a diacrylate, dimethacrylate, or methacrylate-acrylate represented by Formula (I) above. The curable composition containing a diacrylate as the component (A-1) will tend to form a cured product in which a photochromic dye can have a high color fading speed. The curable composition containing a dimethacrylate as the component (A-1) will tend to form a cured product in which a photochromic dye can have a high color optical density. R¹ and R⁷ are each preferably a methyl group.

In Formula (I), R⁴ is an optionally substituted, linear or branched alkylene group having 1 to 7 carbon atoms,
c1 is 2 or more and 100 or less, and
c1 is a number greater than a1, greater than b1, greater than d1, and greater than e1.

Specifically, the repeating unit -(OCH₂CH₂R⁴)- with the subscript c1 corresponds to the first alkylene oxide unit having 4 or more and 9 or less carbon atoms. The polymer moiety including this repeating unit can form a soft segment in a cured product. R⁴ is preferably a linear alkylene group. The alkylene group preferably 1 or more and 4 or less carbon atoms, more preferably 2 or more and 4 or less carbon atoms. The component (A-1) with the alkylene group having a larger number of carbon atoms can form a cured product with higher functionality. However, the component (A-1) with the alkylene group having too large a number of carbon atoms may provide a reduced amount of soft segments per unit mass and thus may form a cured product with reduced functionality.

For good balance between functionality and hardness, c1 is preferably 2 or more and 85 or less, more preferably 2 or more and 70 or less, even more preferably 3 or more and 50 or less, furthermore preferably 5 or more and 45 or less.

R², R³, R⁵, and R⁶ are each independently a hydrogen atom or an alkyl group having 1 to 3 carbon atoms. Preferably, R², R³, R⁵, and R⁶ are each independently a hydrogen atom or a methyl group. R² and R³ are different groups. R⁵ and R⁶ are different groups. R² and R⁶ may be the same group. R³ and R⁵ may be the same group.

In Formula (I), a1 and e1 are each 0 or more and 10 or less. For good balance between functionality and hardness, a1 and e1 are each preferably 0 or more and 5 or less, more preferably 0 or more and 2 or less, even more preferably 0 or 1, most preferably 0.

In Formula (I), b1 and d1 are each 0 or more and 20 or less. For good balance between functionality and hardness, b1 and d1 are each preferably 0 or more and 15 or less, more preferably 0 or more and 10 or less, even more preferably 0 or more and 5 or less, furthermore preferably 0.

Specifically, the component (A-1) may be a monomer having at least one selected from the group consisting of the second alkylene oxide unit, which is the repeating unit with the subscript b1 or d1, and the third alkylene oxide unit, which is the repeating unit with the subscript a1 or e1.

The component (A-1) is preferably represented by Formula (I) with a1, b1, d1, and e1 being 0. In other words, the component (A-1) preferably has only the first alkylene oxide unit. The component (A-1) with such a feature will tend to form a cured product with higher hardness. In Formula (I), c1 may be 4 or more and 20 or less or 6 or more and 15 or less.

For example, the component (A-1) with such a feature may be represented by Formula (3) below.

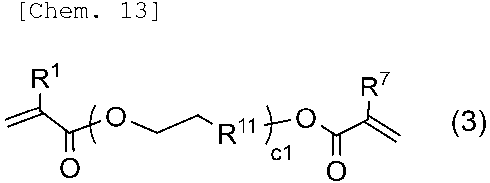

In Formula (3) above, R¹, R⁷, and c1 are as defined for Formula (I).

R¹¹ is a linear alkylene group having 1 to 7 carbon atoms. R¹¹ is preferably a linear alkylene group having 1 to 5 carbon atoms, more preferably a linear alkylene group having 1 to 3 carbon atoms, most preferably a linear alkylene group having 2 carbon atoms.

Examples of the compound represented by Formula (3) above include polytrimethylene glycol di(meth)acrylate, polytetramethylene glycol di(meth)acrylate, polypentamethylene glycol di(meth)acrylate, and polyhexamethylene glycol di(meth)acrylate.

For good balance between photochromic properties and hardness and for the curable composition to have desired viscosity, the component (A-1) represented by Formula (3) preferably has a number-average molecular weight of 200 or more and 9,000 or less, more preferably 200 or more and 7,000 or less, even more preferably 200 or more and 6,000 or less, most preferably 250 or more and 5,000 or less. The component (A-1) may have a number-average molecular weight of 400 or more and 1,500 or less or 600 or more and 1,000 or less.

The component (A-1) represented by Formula (I) with a1 and e1 being 0 and b1 and d1 being 1 or more (namely the component (A-1) further having the second alkylene oxide unit) will tend to form a cured product that allows the functional colorant to exhibit high functionality. In Formula (I), b1 and d1 may each be 2 or more and 15 or less or 4 or more and 10 or less.

Examples of the component (A-1) with those features include the following.

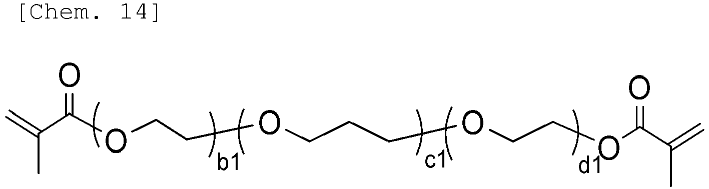

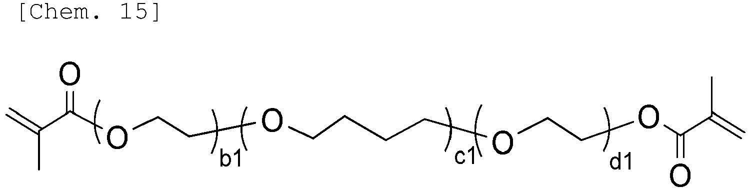

The component (A-1) may also be a monomer represented by Formula (I) with a1, b1, d1, and e1 being 1 or more, namely a monomer having both the second and third alkylene oxide units. In this case, the second and third alkylene oxide units have different structures.

Examples of the component (A-1) with such features include the following.

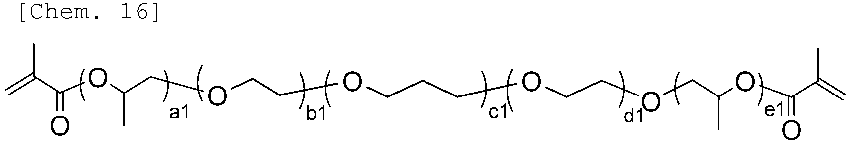

The component (A-1) may be produced, for example, by the method described below.

The component (A-1) having an acryloyl group may be synthesized by esterification of acrylic acid with a polyol compound represented by the formula below. In the formula below for the polyol compound, R², R³, R⁴, R⁵, R⁶, a1, b1, c1, d1, and e1 are as defined for Formula (I).

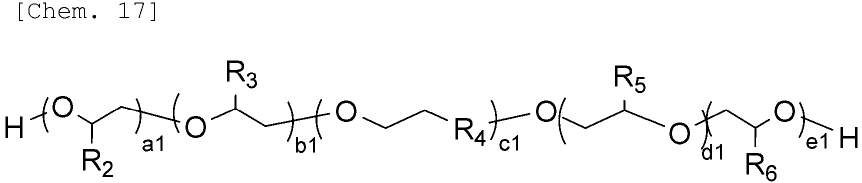

Specifically, the component (A-1) may be synthesized by reacting acrylic acid and the polyol compound dissolved in a solvent, such as toluene, in the presence of a mineral acid, such as sulfuric acid or hydrochloric acid, an organic acid, such as aromatic sulfonic acid, or a Lewis acid, such as boron fluoride ether, while heating and stirring as needed and removing the produced water by azeotropy. In the esterification reaction, the method for removing water may include removing water with a drying agent, such as anhydrous magnesium sulfate or molecular sieves, or removing water in the presence of a dehydrating agent, such as dicyclohexylcarbodiimide.

Alternatively, the component (A-1) may be synthesized by esterification reaction with an acrylic acid halide. Specifically, the esterification reaction may include reacting acrylic acid and the polyol compound dissolved in an ether solvent, such as tetrahydrofuran, in the presence of a base, such as pyridine or dimethylaniline, while heating and stirring as needed; and removing the produced hydrogen halide.

Alternatively, the component (A-1) may be synthesized by ester exchange reaction with an ester compound, such as acrylic anhydride or methyl acrylate. Specifically, the ester exchange reaction may include reacting acrylic acid and the polyol compound dissolved in a solvent, such as toluene, in the presence of an acidic catalyst, such as aromatic sulfonic acid, or a basic catalyst, such as sodium acetate or pyridine, while heating and stirring as needed.

For example, the component (A-1) having a methacryloyl group may be synthesized in the same manner as described above except for using methacrylic acid instead of acrylic acid.

The polyol compound represented by the formula with a1 and c1 being 0 and b1 and d1 being 1 or more (namely the polyol having the second alkylene oxide unit) may be synthesized, for example, by the method described below.

The polyol having the second alkylene oxide unit may be synthesized by the reaction of H-(OCH₂CH₂R⁴)c1-OH with a cyclic ether compound, such as ethylene oxide or propylene oxide. For example, the polyol compound having the second alkylene oxide unit may be synthesized by performing the reaction at high temperature and high pressure in the presence of potassium hydroxide or any other alkali metal hydroxide in an autoclave purged with nitrogen.

The polyol compound represented by the formula with a1, b1, d1, and e1 being 1 or more (namely the component (A-1) further having the second and third alkylene oxide units) may be synthesized, for example, by the method described below.

The component (A-1) further having the second and third alkylene oxide units may be synthesized by a process including: reacting a cyclic ether compound with the polyol compound having the second alkylene oxide unit to form the polyether compound further having the third alkylene oxide unit; and reacting acrylic or methacrylic acid with the resulting polyol compound further having the third alkylene oxide unit in the same manner as described above.

### (A-2): Second Radically Polymerizable Monomer

The curable composition according to an embodiment may further include a second radically polymerizable monomer having three or more (meth)acryloyl groups per molecule. Hereinafter, the second radically polymerizable monomer having three or more (meth)acryloyl groups per molecule will also be referred to as the component (A-2). The curable composition containing such a multifunctional (meth)acrylate will tend to form a cured product with increased hardness.

The component (A-2) may be a multifunctional (meth)acrylate represented by Formula (2) below, a urethane bond-containing multifunctional (meth)acrylate, or any multifunctional (meth)acrylate other than the above, and is preferably a multifunctional (meth)acrylate represented by Formula (2) below.

### Multifunctional (Meth)acrylate Represented by Formula (2)

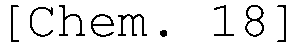

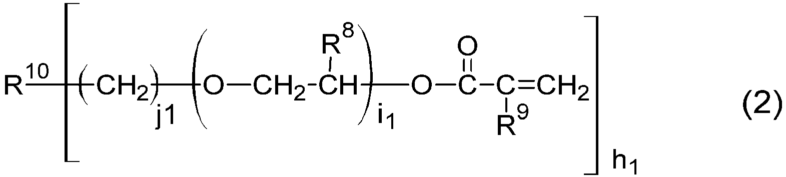

In Formula (2), R⁸ is a hydrogen atom or an alkyl group having 1 to 2 carbon atoms,
R⁸ is preferably a methyl group,
i1 is a number of 0 or more and 3 or less, and
i1 is preferably 0.

In Formula (2), j1 is a number of 0 or more and 3 or less, and
j1 is preferably 0 or 1.

In Formula (2), R⁹ is a hydrogen atom or a methyl group, and
R⁹ is preferably a methyl group.

In Formula (2), R¹⁰ is an organic group having 1 to 10 carbon atoms and having a valence of 3 to 6,
h1 is a number of 3 or more and 6 or less, and
h1 is preferably 3 or more and 5 or less.

The organic group for R¹⁰ may be a polyol-derived group, a hydrocarbon group having a valence of 3 to 6, or a urethane bond-containing organic group having a valence of 3 to 6.

Examples of the multifunctional (meth)acrylate represented by Formula (2) above include trimethylolpropane trimethacrylate, trimethylolpropane triacrylate, glycerol trimethacrylate, glycerol triacrylate, tetramethylolmethane trimethacrylate, tetramethylolmethane triacrylate, tetramethylolmethane tetramethacrylate, tetramethylolmethane tetraacrylate, trimethylolpropane triethylene glycol trimethacrylate, trimethylolpropane triethylene glycol triacrylate, ditrimethylolpropane tetramethacrylate, ditrimethylolpropane tetraacrylate, dipentaerythritol hexaacrylate, dipentaerythritol pentaacrylate, dipentaerythritol hexametacrylate, and dipentaerythritol pentametacrylate.

Urethane Bond-Containing Multifunctional (Meta)acrylate The urethane bond-containing multifunctional (meta) acrylate has a structure different from that of the urethane (meth)arylate represented by Formula (I). The urethane bond-containing multifunctional (meth)acrylate may be a reaction product of a polyisocyanate compound having three or more isocyanate groups per molecule, a polyol compound having two or more hydroxyl groups per molecule, and a hydroxyl group-containing (meth)acrylate. The urethane bond-containing multifunctional (meth)acrylate preferably has four or more (meth)acryloyl groups per molecule. Commercially available examples of the urethane bond-containing multifunctional (meth)acrylate include U-4HA (with a molecular weight of 596 and 4 functional groups), U-6HA (with a molecular weight of 1,019 and 6 functional groups), U-6LPA (with a molecular weight of 818 and 6 functional groups), and U-15HA (with a molecular weight of 2,300 and 15 functional groups) manufactured by Shin-Nakamura Chemical Co., Ltd.

### Multifunctional (Meth)acrylate Other Than the Above

The multifunctional (meth)acrylate other than that represented by Formula (2) and the urethane bond-containing multifunctional (meth)acrylate may be a polyester compound having an end modified with a (meth)acryloyl group. Such a polyester (meth)acrylate compound is commercially available with a variety of (meth)acryloyl modification contents and a variety of molecular weights of the raw material polyester compound. Examples of such a polyester (meth)acrylate compound include tetrafunctional polyester oligomers (with molecular weights of 2,500 to 3,500, such as EB80 manufactured by Daicel-Allnex Ltd.), hexafunctional polyester oligomers (with molecular weights of 6,000 to 8,000, such as EB450 manufactured by Daicel-Allnex Ltd.), hexafunctional polyester oligomers (with molecular weights of 45,000 to 55,000, such as EB1830 manufactured by Daisel-Allnex Ltd.), and tetrafunctional polyester oligomers (specifically with a molecular weight of 10,000, such as GX8488B manufactured by DSK Co., Ltd.).

### (A-3) (Meth)acryloyl Group-Containing Radically Polymerizable Monomer Other Than the Above

The (meth)acryloyl group-containing radically polymerizable monomer other than the above may be a radically polymerizable monomer having a (meth)acryloyl group-containing molecular structure and not corresponding to (A-1) or (A-2). Such a radically polymerizable monomer may be any type known in the art, examples of which include a third radically polymerizable monomer having only one (meth)acryloyl group, a fourth radically polymerizable monomer having a structure different from that of the first radically polymerizable compound and having a difunctional (meth)acryloyl group, and a fifth radically polymerizable monomer having a structure different from those of the first to fourth radically polymerizable monomers and having at least one (meth)acryloyl group per molecule.

### Third Radically Polymerizable Monomer

The curable composition according to an embodiment may further include a third radically polymerizable monomer having one (meth)acryloyl group per molecule. The third radically polymerizable monomer may be a monofunctional (meth)acrylate.

The monofunctional (meth)acrylate may be one represented by Formula (4) below.

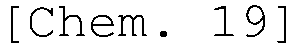

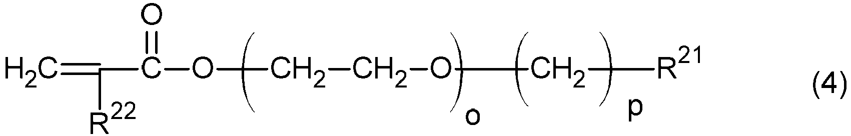

In Formula (4), R²¹ is a hydrogen atom, a methyldimethoxysilyl group, a trimethoxysilyl group, a glycidyl group, a pentamethylpiperidino group, or a 2,2,6,6-tetramethylpiperidino group,
R²² is a hydrogen atom or a methyl group,
o is an integer of 0 to 10, and
p is an integer of 0 to 20.

R²¹ is preferably a methyldimethoxysilyl group, a trimethoxysilyl group, or a glycidyl group. The curable composition containing the monofunctional acrylate with such a functional group will tend to form a cured product having improved adhesion to a base material.

Examples of the monofunctional (meth)acrylate represented by Formula (4) above include methoxypolyethylene glycol methacrylate, methoxypolyethylene glycol acrylate, stearyl methacrylate, lauryl methacrylate, methyl acrylate, ethyl acrylate, butyl acrylate, octyl acrylate, lauryl acrylate, γ-methacryloyloxypropyltrimethoxysilane, γ-methacryloyloxypropylmethyldimethoxysilane, glycidyl methacrylate, 1,2,2,6,6-pentamethyl-4-piperidyl methacrylate, and 2,2,6,6-tetramethyl-4-piperidyl methacrylate.

### Difunctional (Meth)acrylate Having Two (Meth)acryloyl Groups per Molecule

In addition to the first radically polymerizable monomer, the curable composition according to an embodiment may contain the fourth radically polymerizable monomer having a difunctional (meth)acryloyl groups as shown below. The addition of the fourth radically polymerizable monomer can enhance the functionality of the functional colorant. Specifically, the fourth radically polymerizable monomer may be a difunctional (meth)acrylate represented by Formula (5), (6), or (7) below, a urethane bond-containing, difunctional (meth)acrylate, or any difunctional (meth)acrylate other than the above.

### Difunctional (Meth)acrylate Compound Represented by Formula (5)

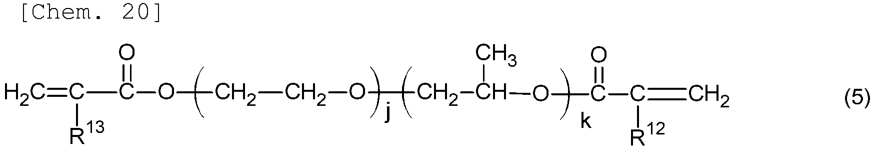

In Formula (5), R¹² and R¹³ are each a hydrogen atom or a methyl group, and
j and k are each independently an integer of 0 or more, and j + k is an integer of 2 or more. In many cases, the difunctional (meth)acrylate compound represented by Formula (5) is produced in the form of a mixture. Thus, j + k may be an integer of 2 or more on average, and is preferably an integer of 2 or more and 50 or less on average.

Examples of the compound represented by Formula (5) above may be as follows:
Diethylene glycol dimethacrylate, triethylene glycol dimethacrylate, tetraethylene glycol dimethacrylate, pentaethylene glycol dimethacrylate, pentapropylene glycol dimethacrylate, diethylene glycol diacrylate, triethylene glycol diacrylate, tetraethylene glycol diacrylate, pentaethylene glycol diacrylate, tripropylene glycol diacrylate, tetrapropylene glycol diacrylate, pentapropylene glycol diacrylate, dimethacrylates of a mixture of polypropylene glycol and polyethylene glycol, polyethylene glycol dimethacrylate, tripropylene glycol dimethacrylate, tetrapropylene glycol dimethacrylate, polypropylene glycol dimethacrylate, polyethylene glycol diacrylate, polyethylene glycol diacrylate, or polyethylene glycol methacrylate acrylate.

### Difunctional (Meth)acrylate Represented by Formula (6)

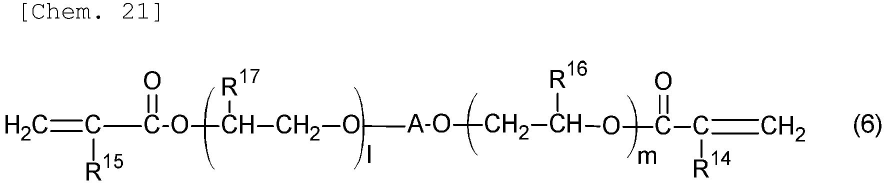

In Formula (6), R¹⁴ and R¹⁵ are each a hydrogen atom or a methyl group, and
R¹⁶ and R¹⁷ are each a hydrogen atom or a methyl group.

In Formula (6), A is a divalent organic group. In Formula (6), A may be a linear or branched alkylene group having 1 to 20 carbon atoms, a phenylene group optionally substituted with halogen or an alkyl group having 1 to 5 carbon atoms, a cycloalkylene group, a bicycloalkylene group, a tricycloalkylene group, or any one of the groups represented by the following formulas.

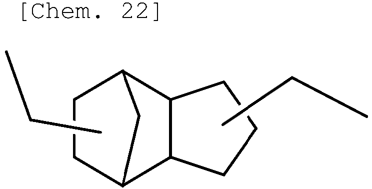

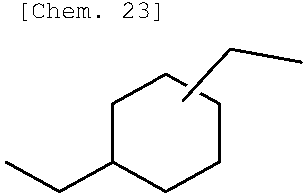

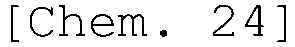

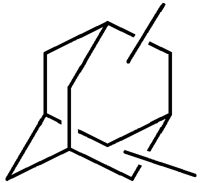

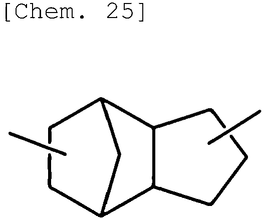

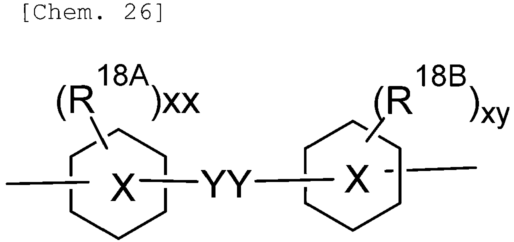

In the formula, R^{18A} and R^{18B} are each a hydrogen atom, an alkyl group having 1 to 5 carbon atoms, or a halogen atom, xx and xy are each 0 to 4,
the ring X is a benzene ring or a cyclohexane ring, and YY is -O-, -S-, -(SO₂)-, -CO-, -CH₂-, -CH=CH-, -C(CH₃)₂-, - C(CH₃)(C₆H₅)-, or any one of the following groups:

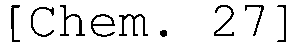

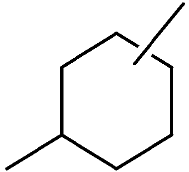

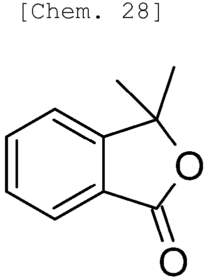

In Formula (6), 1 and m are each an integer of 1 or more, and 1 + m is 2 or more and 30 or less on average.

Examples of the difunctional (meth)acrylate represented by Formula (6) above include bisphenol A di(meth)acrylate, 2,2-bis[4-(methacryloyloxyethoxy)phenyl]propane, 2,2-bis[3,5-dibromo-4-(methacryloyloxyethoxy)phenyl]propane, 2,2-bis[4-(methacryloyloxydipropoxy)phenyl]propane, 2,2-bis[4-(acryloyloxydiethoxy)phenyl]propane, 2,2-bis[4-(acryloyloxypolyethoxy)phenyl]propane, 2,2-bis[4-(methacryloyloxypolyethoxy)phenyl]propane, 1,3-adamantanediol dimethacrylate, and ditrimethylolcyclodecane diacrylate.

### Difunctional (Meth)acrylate Represented Formula (7)

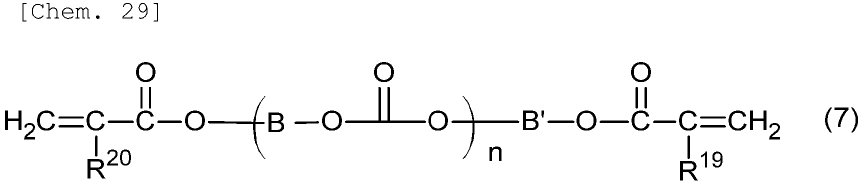

In Formula (7), R¹⁹ and R²⁰ are each a hydrogen atom or a methyl group.

In Formula (7), n is a number of 1 or more and 20 or less on average.

In Formula (7), B and B' are each independently a linear or branched alkylene group having 2 to 15 carbon atoms, B and B' may be the same or different, and
when two or more B are present, the two or more B may be the same group or different groups.

The difunctional (meth)acrylate represented by Formula (7) above may be produced by the reaction of a polycarbonate diol with (meth)acrylic acid.

The polycarbonate diol for use in this reaction may be any of those listed below. Examples include polycarbonate diols (with average molecular weights of 500 to 2,000) obtained by the phosgenation of trimethylene glycol, polycarbonate diols (with average molecular weights of 500 to 2,000) obtained by the phosgenation of tetramethylene glycol, polycarbonate diols (with average molecular weights of 500 to 2,000) obtained by the phosgenation of pentamethylene glycol, polycarbonate diols (with average molecular weights of 500 to 2,000) obtained by the phosgenation of hexamethylene glycol, polycarbonate diols (with average molecular weights of 500 to 2,000) obtained by the phosgenation of octamethylene glycol, polycarbonate diols (with average molecular weights of 500 to 2,000) obtained by the phosgenation of nonamethylene glycol, polycarbonate diols (with average molecular weights of 500 to 2,000) obtained by the phosgenation of triethylene glycol and tetramethylene glycol, polycarbonate diols (with average molecular weights of 500 to 2,000) obtained by the phosgenation of tetramethylene glycol and hexamethylene glycol, polycarbonate diols (with average molecular weights of 500 to 2,000) obtained by the phosgenation of pentamethylene glycol and hexamethylene glycol, polycarbonate diols (with average molecular weights of 500 to 2,000) obtained by the phosgenation of tetramethylene glycol and octamethylene glycol, polycarbonate diols (with average molecular weights of 500 to 2,000) obtained by the phosgenation of hexamethylene glycol and octamethylene glycol, and polycarbonate diols (with average molecular weights of 500 to 2,000) obtained by the phosgenation of 1-methyltrimethylene glycol.

### Urethane Bond-Containing Difunctional (Meth)acrylate

The urethane bond-containing difunctional (meth)acrylate may be a reaction product of a polyisocyanate compound having two or more isocyanate groups per molecule, a polyol compound having two or more hydroxyl groups per molecule, and a hydroxyl group-containing (meth)acrylate.

For example, the polyisocyanate is preferably hexamethylene diisocyanate, isophorone diisocyanate, lysine isocyanate, 2,2,4-hexamethylene diisocyanate, dimer acid diisocyanate, isopropylidene-bis-4-cyclohexyl isocyanate, dicyclohexylmethane diisocyanate, norbornene diisocyanate, or methylcyclohexane diisocyanate.

The polyol may be a polyalkylene glycol having a C2 to C4, ethylene oxide, propylene oxide, or hexamethylene oxide repeating unit or a polyester diol, such as polycaprolactone diol. The polyol may also be polycarbonate diol, polybutadiene diol, pentaerythritol, ethylene glycol, propylene glycol, 1,3-propanediol, 1,4-butanediol, 1,5-pentanediol, 1,6-hexanediol, 1,9-nonanediol, 1,8-nonanediol, neopentyl glycol, diethylene glycol, dipropylene glycol, 1,4-cyclohexanediol, 1,4-cyclohexanedimethanol, glycerin, or trimethylolpropane.

The urethane bond-containing difunctional (meth)acrylate material may also be a reaction mixture obtained by the reaction of 2-hydroxy(meth)acrylate with a urethane prepolymer (a reaction product of the polyisocyanate and the polyol) or a reaction mixture including urethane (meth)acrylate monomers obtained by the direct reaction of the diisocyanate with 2-hydroxy(meth)acrylate.

The hydroxyl group-containing (meth)acrylate may be, for example, 2-hydroxyethyl (meth)acrylate, 2-hydroxypropyl (meth)acrylate, 3-hydroxypropyl (meth)acrylate, 3-hydroxybutyl (meth)acrylate, or 4-hydroxybutyl (meth)acrylate.

The urethane bond-containing difunctional (meth)acrylate may be any commercially available product, examples of which include U-2PPA (with a molecular weight of 482), UA-122P (with a molecular weight of 1,100), and U-122P (with a molecular weight of 1,100) manufactured by Shin-Nakamura Chemical Co., Ltd., and EB4858 (with a molecular weight of 454) manufactured by Daicel-Allnex Ltd.

### Difunctional (Meth)acrylate Other Than the Above

The curable composition may contain a sulfur atom-containing difunctional (meth)acrylate. The sulfur atom preferably forms a sulfide group in its molecular chain. Examples include bis(2-methacryloyloxyethylthioethyl) sulfide, bis(methacryloyloxyethyl) sulfide, bis(acryloyloxyethyl) sulfide, 1,2-bis(methacryloyloxyethylthio)ethane, 1,2-bis(acryloyloxyethyl)ethane, bis(2-methacryloyloxyethylthioethyl) sulfide, bis(2-acryloyloxyethylthioethyl) sulfide, 1,2-bis(methacryloyloxyethylthioethylthio)ethane, 1,2-bis(acryloyloxyethylthioethylthio)ethane, 1,2-bis(methacryloyloxyisopropylthioisopropyl) sulfide, and 1,2-bis(acryloyloxyisopropylthioisopropyl) sulfide.

The curable composition may contain one difunctional (meth)acrylate compound of any one of the types described above or two or more difunctional (meth)acrylate compounds of any one of the types described above. The curable composition may also contain a combination of two or more of the types described above. When the curable composition contains a combination of two or more compounds of two or more types, the total mass of those compounds should be determined as a basis for the calculation of the component content.

### Additional Radically Polymerizable Monomer

The curable composition according to an embodiment may further contain an additional radically polymerizable monomer as a fifth radically polymerizable monomer. The additional radically polymerizable monomer may be any known monomer that is radically polymerizable with the component (A-1). The additional radically polymerizable monomer is preferably, for example, a radically polymerizable polyrotaxane, a radically polymerizable silsesquioxane compound, an allyl compound, or a vinyl compound.

### Radically Polymerizable Polyrotaxane

Polyrotaxane has a composite molecular structure consisting of an axle molecule and multiple ring molecules threaded on the axle molecule. It has bulky terminal groups at both ends of the axile molecule, which prevent the ring molecules from dethreading from the axle molecule. The radically polymerizable polyrotaxane has a radically polymerizable group(s) introduced on a side chain(s) of the axle molecule. For example, the radically polymerizable group(s) may be introduced by modifying 1 mol% or more and less than 100 mol% of the hydroxyl groups on the ring molecules to a radically polymerizable group(s). The modification ratio can be calculated as {(the number of moles of polymerizable groups introduced)/(the total number of moles of OH groups on the side chains)} × 100. For good adhesion, high mechanical strength of the cured product, and high functionality, the modification ratio is preferably 10 mol% or more and 95 mol% or less.

The axle molecule with too high a weight-average molecular weight may tend to be less compatible with the other polymerizable monomers. The axle molecule with too low a weight-average molecular weight may tend make the ring molecules less movable. The axle molecule preferably has a weight-average molecular weight in the range of 1,000 to 100,000, more preferably in the range of 5,000 to 80,000, most preferably in the range of 8,000 to 50,000.

The ring molecule is preferably a cyclodextrin ring, a crown ether ring, a benzo-crown ring, a dibenzo-crown ring, or a dicyclohexano-crown ring, more preferably a cyclodextrin ring or a crown ether ring, most preferably a cyclodextrin ring. Cyclodextrin rings include α ring (with a ring inner diameter of 0.45 to 0.6 nm), β ring (with a ring inner diameter of 0.6 to 0.8 nm), and γ ring (with a ring inner diameter of 0.8 to 0.95 nm), among which the α- and β-cyclodextrin rings are preferred, and the α-cyclodextrin ring is most preferred. If the number of fully threaded ring molecules on the axle molecule is normalized to 1, the number of practically threaded ring molecules should preferably be in the range of 0.001 to 0.6, more preferably in the range of 0.002 to 0.5, most preferably in the range of 0.003 to 0.4.

For good reactivity with the other polymerizable monomers and other desired properties, the radically polymerizable group is preferably a (meth)acryloyl group. The number of radically polymerizable groups in the polyrotaxane compound is preferably, but not limited to, 0 to 5,000 per molecule.

Such (meth)acryloyl group-containing polyrotaxanes are described in PCT International Publication No. WO2018/030257.

### Radically Polymerizable Silsesquioxane Compound

The radically polymerizable silsesquioxane compound has a radically polymerizable group, such as a (meth)acrylic group, and may have a variety of molecular structures, such as cage, ladder, and random molecular structures.

Examples of such polymerizable silsesquioxane compounds include compounds represented by Formula (8) below.

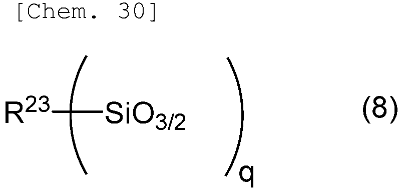

In Formula (8), q is the degree of polymerization and is an integer of 3 to 100.

In Formula (8), two or more R²³ may be the same or different, and are each a radically polymerizable group, a radically polymerizable group-containing organic group, a hydrogen atom, an alkyl group, a cycloalkyl group, an alkoxy group, or a phenyl group, and at least one of R²³ is a radically polymerizable group or a radically polymerizable group-containing organic group.

The radically polymerizable group or radically polymerizable group-containing organic group for R²³ may be a (meth)acrylic group; a (meth)acryloyloxypropyl group, a (3-(meth)acryloyloxypropyl)dimethylsiloxy group, or any other (meth)acrylic group-containing organic group; an allyl group; an allylpropyl group, an allylpropyldimethylsiloxy group, or any other allyl group-containing organic group; a vinyl group; a vinylpropyl group, a vinyldimethylsiloxy group, or any other vinyl group-containing organic group.

### Polymerizable Allyl Compound

Examples of the polymerizable ally compound, which has an allyl group, may be as follows:
Diethylene glycol bisallyl carbonate, methoxypolyethylene glycol allyl ether, methoxypolyethylene glycol-polypropylene glycol allyl ether, butoxypolyethylene glycol-polypropylene glycol allyl ether, phenoxypolyethylene glycol allyl ether, vinyloxypolyethylene glycol allyl ether, styryloxypolyethylene glycol allyl ether, and methoxypolyethylene thioglycol allyl thioether.

### Polymerizable Vinyl Compound

Examples of the polymerizable vinyl compound, which has a vinyl group, include methyl vinyl ketone, ethyl vinyl ketone, ethyl vinyl ether, styrene, vinylcyclohexane, butadiene, 1,4-pentadiene, divinyl sulfide, divinyl sulfone, 1,2-divinylbenzene, 1,3-divinyl-1,1,3,3-tetramethylpropanedisiloxane, diethylene glycol divinyl ether, divinyl adipate, divinyl sebacate, ethylene glycol divinyl ether, divinyl sulfoxide, divinyl persulfide, dimethyldivinylsilane, 1,2,4-trivinylcyclohexane, methyltrivinylsilane, α-methyl styrene, and α-methyl styrene dimer.

### Component Content of Curable Composition

The content of the (meth)acrylate of Formula (1A) in the curable composition according to an embodiment may be, for example, 5 mass% or more and 99 mass% or less. The curable composition with a high content of the (meth)acrylate of Formula (1A) will tend to form a cured product that allows the functional colorant to exhibit enhanced performance. The content of the (meth)acrylate of Formula (1A) may be 15 mass% or more, preferably 20 mass% or more, more preferably 25 mass% or more, even more preferably 30 mass% or more, furthermore preferably 35 mass% or more. However, the curable composition with too high a content of the (meth)acrylate of Formula (1A) may tend to form a cured product with reduced hardness. The content of the (meth)acrylate of Formula (1A) is preferably 90 mass% or less, more preferably 80 mass% or less.

The content of the first radically polymerizable monomer in the curable composition according to an embodiment is preferably 15 mass% or more. The curable composition with a high content of the first radically polymerizable monomer will tend to form a cured product that allows the functional colorant to exhibit enhanced performance. The content of the first radically polymerizable monomer is more preferably 20 mass% or more, even more preferably 30 mass% or more. However, the curable composition with too high a content of the first radically polymerizable monomer may tend to form a cured product with reduced hardness. The content of the first radically polymerizable monomer is preferably 90 mass% or less, more preferably 80 mass% or less.

To form a cured product with high hardness, the curable composition according to an embodiment preferably further contains the second radically polymerizable monomer. The content of the second radically polymerizable monomer in the curable composition is preferably 1 mass% or more, more preferably 10 mass% or more, even more preferably 20 mass% or more, furthermore preferably 25 mass% or more. However, the curable composition with too high a content of the second radically polymerizable monomer may tend to form a cured product in which the functional colorant may exhibit reduced performance. The content of the second radically polymerizable monomer is preferably 85 mass^{®} or less, more preferably 65 mass% or less, even more preferably 55 mass% or less.

To have adjustable viscosity and high compatibility, the curable composition according to an embodiment preferably contains the fourth radically polymerizable monomer. The content of the fourth radically polymerizable monomer in the curable composition is preferably 3 mass% or more, more preferably 5 mass% or more, even more preferably 10 mass% or more. However, the curable composition with too high a content of the fourth radically polymerizable monomer may tend to form a cured product in which the functional colorant may exhibit reduced performance. The content of the fourth radically polymerizable monomer is preferably 60 mass% or less, more preferably 50 mass% or less, even more preferably 40 mass% or less.

To have adjustable viscosity and to form a cured product with high adhesion, the curable composition according to an embodiment preferably contains the third radically polymerizable monomer. The content of the third radically polymerizable monomer in the curable composition is preferably 0.1 mass% or more, more preferably 1 mass% or more, even more preferably 3 mass% or more. However, the curable composition with too high a content of the third radically polymerizable monomer may tend to form a cured product in which the functional colorant may exhibit reduced performance. The content of the third radically polymerizable monomer is preferably 20 mass% or less, more preferably 10 mass% or less, even more preferably 7 mass% or less.

To have adjustable viscosity and adjustable polymerizability, the curable composition according to an embodiment preferably contains the fifth radically polymerizable monomer. The content of the fifth radically polymerizable monomer in the curable composition is preferably 0.1 mass% or more, more preferably 0.5 mass% or more, even more preferably 1 mass% or more. However, the curable composition with too high a content of the fifth radically polymerizable monomer may tend to form a cured product in which the functional colorant may exhibit reduced performance. The content of the fifth radically polymerizable monomer is preferably 15 mass% or less, more preferably 10 mass% or less, even more preferably 7 mass% or less.

The curable composition containing the first, second, third, fourth, and fifth radically polymerizable monomers may have a first radically polymerizable monomer content of 40 mass% or more and 80 mass% or less, a second radically polymerizable monomer content of 10 mass% or more and 40 mass% or less, a third radically polymerizable monomer content of 1 mass% or more and 10 mass% or less, a fourth radically polymerizable monomer content of 5 mass% or more and 50 mass% or less, and a fifth radically polymerizable monomer content of 1 mass% or more and 10 mass% or less, and may contain the functional colorant and any additive as the remainder.

The curable composition according to an embodiment preferably has a methacrylate content of 50 mass% or more. The curable composition with a high methacrylate content will tend to form a cured product that allows the functional colorant to exhibit high functionality. The methacrylate content of the curable composition is preferably 60 mass% or more, more preferably 70 mass% or more, even more preferably 90 mass% or more. The methacrylate content may have an upper limit of 99 mass% or less in an example and may have an upper limit of 95 mass% or less in another example.

The curable composition according to an embodiment may have a ratio of M100 to M101 (M100/M101 ratio) of 0.1 or more and 20 or less, 0.5 or more and 10 or less, or 1 or more and 8 or less, in which M100 is the mass of the methacrylate, and M101 is the mass of the acrylate. As used herein, the term "methacrylate" means the compound having a methacryloyl group and not having any acryloyl group in the curable composition. The term "acrylate" means the compound having an acryloyl group and not having any methacryloyl group in the curable composition.

When the (meth)acrylate represented by Formula (1A), the component (A-1), the component (A-2), and the component (A-3) are collectively referred to as the component (A), the curable composition may contain 15 to 100 parts by mass, 20 to 90 parts by mass, 30 to 80 parts by mass, or 35 to 65 parts by mass of the (meth)acrylate based on 100 parts by mass of the component (A).

The curable composition may contain 15 to 85 parts by mass, 20 to 80 parts by mass, 30 to 70 parts by mass, or 35 to 65 parts by mass of the component (A-1) based on 100 parts by mass of the component (A).

The curable composition may contain 1 to 85 parts by mass, 3 to 75 parts by mass, 5 to 70 parts by mass, or 10 to 65 parts by mass of the component (A-2) based on 100 parts by mass of the component (A).

The curable composition may contain 0.01 to 20 parts by mass, 0.1 to 17 parts by mass, or 0.5 to 15 parts by mass of the component (A-3) based on 100 parts by mass of the component (A).

### (B) Functional Colorant

The functional colorant includes a compound that has the ability to selectively absorb visible rays and develops color, becomes colorless, or changes color upon exposure to light, heat, electric field, pressure, or any other type of energy. The functional colorant with such features changes its structure under specific conditions to provide a specific function. The functional colorant includes, for example, at least one selected from the group consisting of a photochromic compound, an ultraviolet absorber, a blue light absorber, an infrared absorber, and an electrochromic compound.

For example, the curable composition may have an M1/M2 ratio of 1 or more and 10,000 or less, in which M1 is the mass of the (meth)acrylate represented by Formula (1A), and M2 is the mass of the functional colorant. The M1/M2 ratio is preferably 1 or more and 2,500 or less, more preferably 5 or more and 1,000 or less, even more preferably 5 or more and 1,000 or less.

The content of the functional colorant in the curable composition is, for example, 0.001 mass% or more and 10 mass% or less. The content of the functional colorant is preferably 0.1 mass% or more and 8 mass% or less, more preferably 1 mass% or more and 5 mass% or less.

### Photochromic Compound

The curable composition may contain a photochromic compound in an amount enough to exhibit desired photochromic properties. The curable composition preferably contains 0.001 to 10 parts by mass of a photochromic compound based on 100 parts by mass of the component (A).

The photochromic compound content is preferably adjusted to an optimal content depending on the intended use.

Specifically, the curable composition containing the photochromic compound may be used to form a thin coating film, such as a thin film with a thickness of about 100 µm (a polymer film formed through the polymerization of the photochromic curable composition). In such a case, the curable composition preferably contains 0.1 to 10 parts by mass of the photochromic compound for color adjustment based on 100 parts by mass of the polymerizable compound(s).

Alternatively, the curable composition may be used to form a thick cured product (a formed polymer product produced through the polymerization of the photochromic curable composition), such as a cured product with a thickness of 1 mm or more. In such a case, the curable composition preferably contains 0.001 to 1 parts by mass of the photochromic compound for color adjustment based on 100 parts by mass of the polymerizable compound(s) for forming the thick cured product.

The curable composition may contain any known photochromic compound. The curable composition may contain a single photochromic compound or a combination of two or more photochromic compounds. The photochromic compound is typically a chromene compound, a fulgide compound, a fulgimide compound, or a spirooxazine compound. Such compounds are disclosed in many literatures, such as Japanese Unexamined Patent Application, Publication Nos. H2-28154 and S62-288830 and PCT International Publication Nos. WO94/22850, WO96/14596, WO2022/075330, and WO2022/168989.

In particular, the photochromic compound is preferably a chromene compound or a spirooxazine compound. The photochromic compound is more preferably a chromene compound. Chromene compounds include 1-benzopyran skeleton-containing compounds, spiropyran compounds, which have a spiropyran skeleton, and naphthopyran compounds, which have a naphthopyran skeleton.

Naphthopyran compounds preferably include compounds represented by Formulas (9), (10), (11), (12), (13), and (14) below.

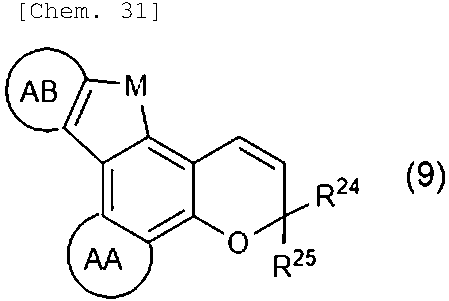

In Formula (9), ring AA is a substituted or unsubstituted aromatic hydrocarbon ring, a substituted or unsubstituted aromatic heterocyclic ring, or a substituted or unsubstituted condensed polycyclic ring having an aromatic ring or aromatic heterocyclic ring condensed with any one of the foregoing rings, and
ring AA may be absent.

In Formula (9), ring AB is a substituted or unsubstituted aromatic hydrocarbon ring, a substituted or unsubstituted aromatic heterocyclic ring, or a substituted or unsubstituted condensed polycyclic ring having an aromatic ring or aromatic heterocyclic ring condensed with any one of the foregoing rings.

In Formula (9), R²⁴ and R²⁵ are each independently a hydrogen atom or a substituent, and two or more substituents may be linked to form a ring structure.

The substituent is preferably at least one group selected from the group consisting of hydroxyl, alkyl, haloalkyl, cycloalkyl, alkoxy, alkoxyalkyl, formyl, hydroxycarbonyl, alkylcarbonyl, alkoxycarbonyl, halogen, optionally substituted aralkyl, optionally substituted aralkoxy, optionally substituted aryloxy, alkylthio, optionally substituted arylthio, optionally substituted aryl, amino, substituted amino, optionally substituted heterocyclic group, haloalkylthio, optionally substituted cycloalkylthio, oligomer group, and a group represented by Formula (15) below.

-Q¹-(P¹Q²)ₐₐ-P²Q³ ( 15)

In Formula (15), Q¹ is an alkylene group optionally substituted with a halogen atom,
Q² is an alkylene group optionally substituted with a halogen atom,
Q³ is an alkyl group optionally substituted with a halogen atom,
P¹ and P² are each independently O, S, NR⁷⁰⁰, PR⁷⁰¹, or P(=O), R⁷⁰⁰ is a hydrogen atom, an optionally substituted alkyl group, an optionally substituted cycloalkyl group, an optionally substituted aryl group, or an optionally substituted heteroaryl group,
R⁷⁰¹ is a hydrogen atom, an optionally substituted alkyl group, an optionally substituted cycloalkyl group, an optionally substituted aryl group, or an optionally substituted heteroaryl group, and
aa is 0 or 1 or more and 10 or less.

In Formula (9), M is CR²⁶R²⁷, SiR²⁶R²⁷, GeR²⁶R²⁷, or NR²⁶, and R²⁶ and R²⁷ are each independently a hydrogen atom or a substituent, and two or more substituents may be linked to form a ring structure.

The substituent is preferably at least one group selected from the group consisting of hydroxyl, alkyl, haloalkyl, cycloalkyl, alkoxy, alkoxyalkyl, formyl, hydroxycarbonyl, alkylcarbonyl, alkoxycarbonyl, halogen, optionally substituted aralkyl, optionally substituted aralkoxy, optionally substituted aryloxy, alkylthio, optionally substituted arylthio, optionally substituted aryl, amino, substituted amino, optionally substituted heterocyclic group, and a group represented by Formula (15) above.

When R²⁶ and R²⁷ are linked together to form a ring structure, the ring structure is preferably an aliphatic ring having 3 to 20 ring carbon atoms, a condensed polycyclic ring having an aliphatic ring condensed with an aromatic ring or an aromatic heterocyclic ring, a heterocyclic ring having 3 to 20 ring member atoms, or a condensed polycyclic ring having a heterocyclic ring condensed with an aromatic ring or an aromatic heterocyclic ring.

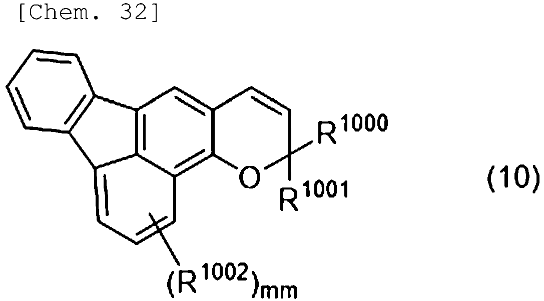

In Formula (10), R¹⁰⁰⁰, R¹⁰⁰¹, and R¹⁰⁰² are each independently a hydrogen atom or a substituent, and two or more substituents may be linked to form a ring structure, the substituent may be the same as that listed for Formula (9), and
mm is 1 to 10.

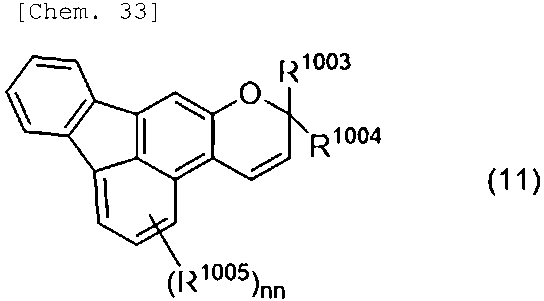

In Formula (11), R¹⁰⁰³, R¹⁰⁰⁴, and R¹⁰⁰⁵ are each independently a hydrogen atom or a substituent, and two or more substituents may be linked to form a ring structure, the substituent may be the same as that listed for Formula (9), and nn is 1 to 10.

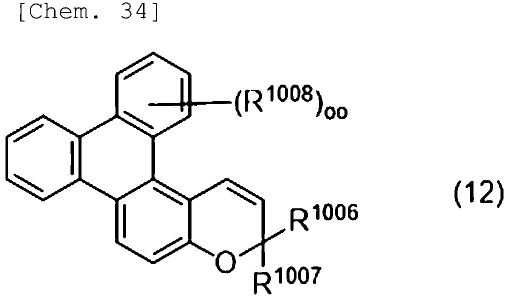

In Formula (12), R¹⁰⁰⁶, R¹⁰⁰⁷, and R¹⁰⁰⁸ are each independently a hydrogen atom or a substituent, and two or more substituents may be linked to form a ring structure, the substituent may be the same as that listed for Formula (9), and
oo is 1 to 12.

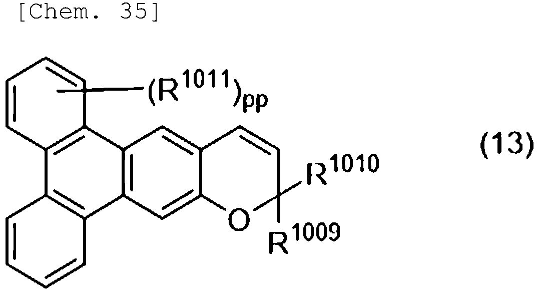

In Formula (13), R¹⁰⁰⁹, R¹⁰¹⁰, and R¹⁰¹¹ are each independently a hydrogen atom or a substituent, and two or more substituents may be linked to form a ring structure, the substituent may be the same as that listed for Formula (9), and
pp is 1 to 12.

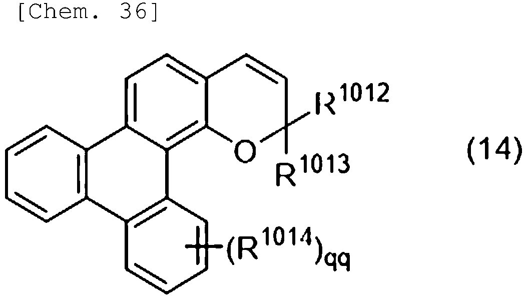

In Formula (14), R¹⁰¹², R¹⁰¹³, and R¹⁰¹⁴ are each independently a hydrogen atom or a substituent, and two or more substituents may be linked to form a ring structure, the substituent may be the same as that listed for Formula (9), and
qq is 1 to 12.

Naphthopyran compounds include indenonaphthopyran compounds, which have an indenonaphthopyran skeleton. The indenonaphthopyran compounds preferably have an indeno[2,1-f]naphtho[1,2-b]pyran skeleton.

For example, the indeno[2,1-f]naphtho[1,2-b]pyran compound may be any one of those disclosed in PCT International Publication Nos. WO1996/014596, WO2001/019813, WO2001/060811, WO2005/028465, WO2006/110221, WO2007/073462, WO2007/140071, WO2008/054942, WO2010/065393, WO2011/10744, WO2011/016582, WO2011/025056, WO2011/034202, WO2011/078030, WO2012/102409, WO2012/102410, and WO2012/121414.

The functional colorant is also preferably a photochromic compound having an oligomer chain group in its molecule. Such an oligomer chain group-containing photochromic compound is disclosed in many literatures, such as PCT International Publication Nos. WO2000/015630, WO2004/041961, WO2009/146509, WO2012/149599, WO2012/162725, WO2013/078086, WO2019/013249, and WO2019/203205. The photochromic compound preferably has an oligomer chain group as disclosed in PCT International Publication No. WO2019/013249 or WO2019/203205 so that it can have high photochromic properties and high durability.

Indenonaphthopyran compounds preferably include compounds represented by Formula (16) below.

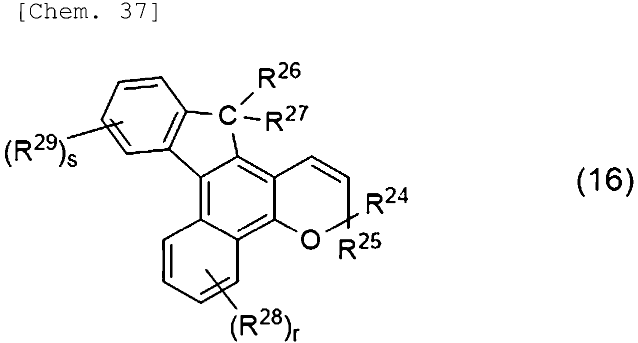

In Formula (16), R²⁴, R²⁵, R²⁶, and R²⁷ are the same as those defined above.

In Formula (16), r is an integer of 0 to 4,
s is an integer of 0 to 4,
when r is 2 to 4, two or more R²⁸ may be the same or different, when s is 2 to 4, two or more R²⁹ may be the same or different, when r is 2 to 4 and when there are adjacent R²⁸, the two adjacent R²⁸ may be liked together with the carbon atom, to which they are attached, to form a ring optionally containing at least one heteroatom selected from the group consisting of oxygen, carbon, sulfur, and nitrogen, and the ring may have a substituent,
when s is 2 to 4 and when there are adjacent R²⁹, the two adjacent R²⁹ may be liked together with the carbon atom, to which they are attached, to form a ring optionally containing at least one heteroatom selected from the group consisting of oxygen, carbon, sulfur, and nitrogen, and the ring may have a substituent.

In Formula (16), R²⁸ and R²⁹ are each independently a group represented by Formula (15), hydroxyl, alkyl, haloalkyl, optionally substituted cycloalkyl, alkoxy, amino, substituted amino, optionally substituted heterocyclic group, halogen, alkylthio, optionally substituted arylthio, nitro, formyl, hydroxycarbonyl, alkylcarbonyl, alkoxycarbonyl, optionally substituted aralkyl, optionally substituted aralkoxy, optionally substituted aryloxy, optionally substituted aryl, optionally substituted heteroaryl, thiol, alkoxyalkylthio, haloalkylthio, optionally substituted cycloalkylthio, optionally substituted silyl, optionally substituted oxysilyl, a group represented by Formula (17) below, or a group represented by L-R⁴⁰⁰.

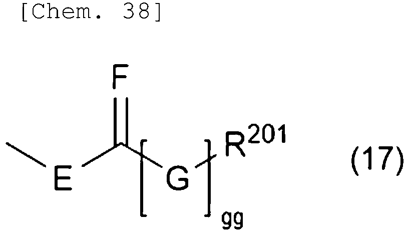

In Formula (17), E is an oxygen atom or NR¹⁰¹, wherein R¹⁰¹ is a hydrogen atom or an alkyl group,
F is an oxygen atom or a sulfur atom,
G is an oxygen atom, a sulfur atom, or NR²⁰²,
R²⁰² is a hydrogen atom, an alkyl group, a cycloalkyl group, an aryl group, or a heteroaryl group,
gg is an integer of 0 or 1,
R²⁰¹ is a hydrogen atom, an alkyl group, a cycloalkyl group, an aryl group, or a heteroaryl group, and
when G is an oxygen atom or a sulfur atom, R²⁰¹ is a group other than a hydrogen atom.

R⁴⁰⁰ is a hydrogen atom, an alkyl group, an aryl group, a substituted silyl group, a polymerizable group, or a photochromic group. The silyl group is substituted with an alkyl group, an alkoxy group, or an aryl group. L is a group represented by Formula (18) below.

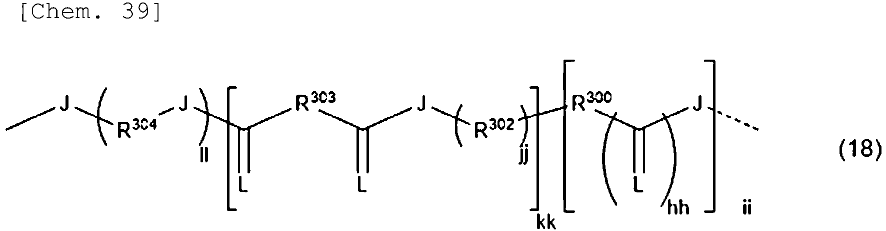

In Formula (18), J is a divalent group and is independently a direct bond, a substituted methylene group, an oxygen atom, a sulfur atom, or NR³⁰¹,
R³⁰¹ is a hydrogen atom or an alkyl group,
L is an oxygen atom or a sulfur atom,
R³⁰⁰ is an alkylene group or a silylene group substituted with an alkyl group or an aryl group,
R³⁰², R³⁰³, and R³⁰⁴ are each an alkylene group,
hh, jj, kk, and 11 are each an integer of 0 or 1,
ii is an integer of 1 to 200,
two or more units, of which the number is represented by ii, may be the same or different, and
the broken line indicates a bond with R⁴⁰⁰.

### Additional Additive

The curable composition includes the components (A) and (B) as essential components. The curable composition may contain various known additives in amounts that will not compromise the advantageous effect. Such additives include, for example, a mold release agent, an ultraviolet absorber, an infrared absorber, an ultraviolet light stabilizer, an antioxidant, an anti-discoloration agent, an antistatic agent, a fluorescent dye, a dye, a pigment, a fragrance, and other stabilizing agents. The curable composition may also contain a solvent and a leveling agent. The curable composition may also contain a thiol, such as tert-dodecylmercaptan, as a polymerization modifier.

### Ultraviolet Light Stabilizer

The curable composition preferably contains an ultraviolet light stabilizer, which can enhance the durability of the photochromic compound. The ultraviolet light stabilizer is preferably a hindered amine light stabilizer, a hindered phenol antioxidant, or a sulfur-containing antioxidant. For prevention of degradation of the photochromic compound, the hindered amine light stabilizer is preferably, but not limited to, bis(1,2,2,6,6-pentamethyl-4-piperidyl) sebacate. The ultraviolet light stabilizer is also preferably a hindered amine light stabilizer commercially available under the trade name of ADEKA STAB LA-52, LA-57, LA-62, LA-63, LA-67, LA-77, or LA-87 from ADEKA Corporation.

The hindered phenol antioxidant is preferred for prevention of degradation of the photochromic compound. Examples of the hindered phenol antioxidant include 2,6-ditert-butyl-4-methyl-phenol, IRGANOX 245 manufactured by BASF Japan Ltd. (ethylenebis(oxyethylene)bis[3,5-tert-butyl-4-hydroxy-m-tolyl]propionate]), IRGANOX 1076 manufactured by BASF Japan Ltd. (octadecyl-3-(3,5-di-tert-butyl-4-hydroxyphenyl)propionate), IRGANOX 1010 manufactured by BASF Japan Ltd. (pentaerythritol tetrakis[3-(3,5-di-tert-butyl-4-hydroxyphenyl)propionate]), and IRGANOX 1035, 1075, 1098, 1135, 1141, 1222, 1330, 1425, 1520, 259, 3114, 3790, 5057, and 565 manufactured by BASF Japan Ltd. The curable composition may contain such an ultraviolet light stabilizer in any amount that will not compromise the advantageous effect. In general, the content of such an ultraviolet light stabilizer in the curable composition is in the range of 0.001 to 10 parts by mass, preferably in the range of 0.01 to 1 part by mass, based on 100 parts by mass of the curable composition.

### Polymerization Initiator

Polymerization initiators include thermal polymerization initiators and photopolymerization initiators. Examples are as shown below.

Examples of thermal polymerization initiators include
diacyl peroxides (e.g., benzoyl peroxide, p-chlorobenzoyl peroxide, decanoyl peroxide, lauroyl peroxide, acetyl peroxide) ;
peroxyesters (e.g., tert-butyl peroxy-2-ethylhexanoate, tert-butyl peroxyneodecanoate, cumyl peroxyneodecanoate, t-butyl peroxybenzoate) ;
percarbonates (e.g., diisopropyl peroxydicarbonate, di-sec-butyl peroxydicarbonate); and
azo compounds (e.g., azobisisobutyronitrile).

Examples of photopolymerization initiators include acetophenone compounds (e.g., 1-phenyl-2-hydroxy-2-methylpropan-1-one, 1-hydroxycyclohexyl phenyl ketone, 1-(4-isopropylphenyl)-2-hydroxy-2-methylpropan-1-one); α-dicarbonyl compounds (e.g., 1,2-diphenylethanedione, methylphenyl glycoxylate); and acylphosphine oxide compounds (e.g., 2,6-dimethylbenzoyldiphenylphosphine oxide, 2,4,6-trimethylbenzoyldiphenylphosphine oxide, methyl 2,4,6-trimethylbenzoyldiphenylphosphinate, 2,6-dichlorobenzoyldiphenylphosphine oxide, 2,6-dimethoxybenzoyldiphenylphosphine oxide).

The curable composition may contain the photopolymerization initiator in combination with a known polymerization curing accelerator, such as a tertiary amine.

### Surfactant

When added to the curable composition, a surfactant can provide it with improved wettability with an optical base material or a primer layer and can prevent it from having degraded appearance. The surfactant may be a known surfactant, such as a silicone surfactant having a silicone chain (polyalkylsiloxane unit) as a hydrophobic group or a fluorosurfactant having a fluorocarbon chain. The curable composition may contain a mixture of two or more surfactants. The surfactant may be or may not be polymerizable with the component (A).

Preferred examples of the silicone surfactant and the fluorosurfactant include L-7001, L-7002, L-7604, FZ-2123, and FZ-2110 manufactured by Dow Toray Co., Ltd., MEGAFACE F-470, MEGAFACE F-1405, and MEGAFACE F-479 manufactured by DIC Corporation, Fluorad FC-430 manufactured by 3M Japan Co., Ltd., TEGORAD 2100 and TEGORAD 2300 manufactured by Evonik Japan Co., Ltd., BYK-UV 3505, BYK-UV 3505, BYK-UV 3510, BYK-UV 3530, BYK-3550, BYK-3560, BYK-UV 3565, BYK-3566, BYK-UV 3500, BYK-UV 3535, BYK-UV 3570, BYK-UV 3575, and BYK-UV 3576 manufactured by BYK Japan K.K., and KR-513, X-22-2445, X-40-9296, X-22-164, X-22-164A, X-22-164B, X-22-164C, and X-22-164E manufactured by Shin-Etsu Chemical Co., Ltd.

### Ultraviolet Absorber

The ultraviolet absorber may be a benzophenone compound, a benzotriazole compound, a cyanoacrylate compound, a triazine compound, a benzoate compound, a cinnamic acid ester compound, an oxanilide compound, or any other known ultraviolet absorber. The ultraviolet absorber is preferably a cyanoacrylate compound, a benzophenone compound, a benzotriazole compound, or a cinnamic acid ester compound. The ultraviolet light stabilizer is preferably added in an amount of 0.001 to 5 parts by mass to 100 parts by mass of the curable composition containing the photochromic compound and the polymerizable compound.

### Cured Product

The cured product is obtained by curing the curable composition. The curable composition may be cured by being subjected to radical polymerization reaction using active energy rays, such as ultraviolet rays, α rays, β rays, γ rays, or LED rays, heat, or a combination of active energy rays and heat. Specifically, appropriate curing methods may be selected depending on the type of the polymerizable monomer or polymerization curing accelerator used or the form of the cured product to be produced. In a case where the laminate described later is formed by a coating method, the curable composition is preferably subjected to photopolymerization so that it can be formed into a uniformly thick film.

The curable composition containing the polymerizable compound may be subjected to thermal polymerization. In such a case, the thermal polymerization temperature may affect the nature of the resulting cured product. The temperature conditions will vary with the type and amount of the thermal polymerization initiator or the type of the polymerizable compound and thus cannot be uniquely determined. In general, however, the thermal polymerization process should preferably be started at relatively low temperatures and performed at gradually increased temperatures. Similar to the polymerization temperature, the polymerization time will also vary with different factors. In general, the time conditions should preferably be selected so that the polymerization can be completed in 2 to 48 hours, although an optimal time should be selected depending on the different conditions. For the formation of a photochromic laminate sheet, an optimal temperature and time should preferably be selected so that the reaction between polymerizable functional groups can proceed to provide the desired molecular weight at the selected temperature.

In the case of photopolymerization of the curable composition, especially UV intensity among polymerization conditions may affect the nature of the photochromic cured product. In general, the photopolymerization conditions should preferably be selected so that the curable composition can be irradiated with UV light with a wavelength of 365 nm at a dose of 50 to 500 mW/cm² for a time period of 0.5 to 5 minutes, although the illuminance conditions will vary with the type and amount of the photopolymerization initiator or the type of the polymerizable monomer and thus cannot be uniquely determined.

### Laminate

Another embodiment provides a laminate. The laminate includes an optical base material and the cured product according to an embodiment provided on a surface of the optical base material. The optical base material includes, for example, a diallyl carbonate resin, a urethane resin, a thiourea resin, or any other resin. The optical base material may be a lens base material. The laminate may include a primer layer between the cured product and the optical base material. The primer layer may include a urethane resin.

FIG. 1 is a schematic cross-sectional view of an example of the laminate according to an embodiment. As shown in FIG. 1, the laminate 10 includes an optical base material 11, a primer layer 1 provided on one main surface of the optical base material 11, and a functional resin layer 12 provided on a main surface of the primer layer 1. The functional resin layer 12 includes the cured product according to an embodiment. The optical base material 11 may be a convex meniscus lens having a convex-concave shape.

### Optical Component

The cured product according to an embodiment can be widely used as an optical component. Examples of such an optical component include various memory materials alternative to silver halide photosensitive materials, copying materials, printing photosensitive materials, memory materials for cathode ray tubes, photosensitive materials for lasers, photosensitive materials for holography, and other various memory materials, and lenses. The lenses are suitable for eyeglasses. The cured product may be a photochromic cured product containing a photochromic compound. Such a photochromic cured product may be used as a photochromic lens material, an optical filter material, a display material, or a material for actinometers, decoration, and other applications.

The cured product according to an embodiment is particularly suitable for photochromic lens applications. Photochromic lenses are suitable for use as eyeglass lenses, such as sunglass lenses. Photochromic lenses may be produced using known methods that can produce uniform photochromic properties.

In a case where a kneading method is used to provide photochromic properties, the optical component may be produced by a process including: casting the curable composition between glass molds held with elastomer gaskets or spacers; and subjecting the curable composition to cast polymerization by heating in an air furnace or by irradiation with active energy rays, such as ultraviolet rays, depending on the type of the polymerizable compound or the polymerization curing accelerator, to form a photochromic cured product in a lens shape or any other optical component shape.

In a case where a lamination method is used to provide photochromic properties, the optical component may be produced by a process including: dissolving the curable composition in an organic solvent as appropriate to form a coating solution; applying the coating solution to the surface of an optical base material, such as a lens base material, by spin coating or dipping; drying the coating to remove the organic solvent; and then subjecting the coating to polymerization and curing by heating, UV irradiation, or other techniques in inert gas, such as nitrogen, to form a photochromic layer including a photochromic cured product on the surface of the optical base material (coating method).

Alternatively, the optical component may be produced by a process including: placing, in a glass mold, an optical base material, such as a lens base material, such that they face each other with a certain space therebetween; casting the curable composition into the space; and subjecting the curable composition in the inner mold to cast polymerization and curing by UV irradiation, heating, or other techniques to form a photochromic layer including a photochromic cured product on the surface of the optical base material (cast polymerization method) .

In a case where a photochromic layer is to be formed on the surface of an optical base material by a lamination method (a coating method or a cast polymerization method) as described above, the surface of the optical base material may be subjected to chemical treatment with an alkali solution or an acid solution or to physical treatment by corona discharge, plasma discharge, or polishing so that the optical base material can have improved adhesion to the photochromic layer. Of course, a transparent adhesive resin layer may be formed on the surface of the optical base material.

The cured product made from the curable composition may also be subjected to additional processing depending on the intended use. Examples of additional processing include dyeing with dyes, such as disperse dyes, formation of a hard coat film using a hard coat agent composed mainly of a silane coupling agent or a silicon, zirconium, antimony, aluminum, tin, or tungsten sol, thin film formation by vapor deposition of metal oxide, such as SiO₂, TiO₂, or ZrO₂, anti-reflection treatment by applying an organic polymer to form a thin film, and antistatic treatment.

### EXAMPLES

Next, the present invention will be described in detail with reference to examples and comparative examples. It will be understood that the examples are not intended to limit the present invention. Materials, evaluation methods, and other techniques used are as described below.

### Synthesis of (Urethane) (meth) acrylate

### Example 1

### Synthesis of PTG25CD100

A methacrylate named PTG25CD100 of the formula below was synthesized by the method described below.

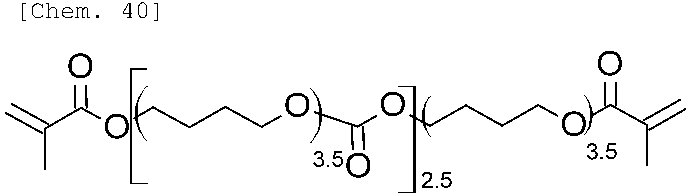

To 100 g of NT1002 (manufactured by Mitsubishi Chemical Corporation) with a molecular weight of 1,000 calculated from the hydroxyl value were added 330 mL of dehydrated toluene, 1.0 mg of p-methoxyphenol, and 2.86 g of p-toluenesulfonic acid monohydrate, and stirred. To the mixture was added 18.9 g of methacrylic acid. The resulting mixture was reacted under azeotropic conditions for 20 hours. After the reaction was completed, 1,000 mL of a 5% sodium hydrogen carbonate aqueous solution was added to the mixture and then subjected to liquid-liquid separation. Celite was added to the resulting organic layer, stirred, and then filtered off. To the resulting organic layer was added 20 g of Wakogel 60N, stirred, and then filtered off. To the resulting organic layer was added 1 mL of a toluene solution of 0.1 mg/mL p-methoxyphenol. The mixture was concentrated to yield PTG25CD100.

The measured proton nuclear magnetic resonance spectrum of PTG25CD100 showed a peak at δ about 1.0 to about 2.5 ppm for about 55 protons of tetramethyleneoxy and methacrylic groups, a peak at δ about 3.0 to about 4.5 ppm for about 49 protons of tetramethyleneoxy groups, and a peak at δ about 5.5 to about 6.5 ppm for 4 protons of acrylic groups.

### Example 2

### Synthesis of PTG25CD200

A methacrylate named PTG25CD200 of the formula below was synthesized by the method described below.

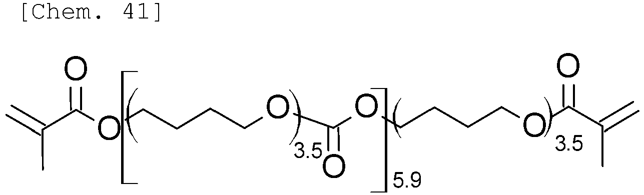

PTG25CD200 was synthesized using the same reaction process as in Example 1 except that NT2002 was used instead of NT1002.

The measured proton nuclear magnetic resonance spectrum of PTG25CD200 showed a peak at δ about 1.0 to about 2.5 ppm for about 103 protons of tetramethyleneoxy and methacrylic groups, a peak at δ about 3.0 to about 4.5 ppm for about 96 protons of tetramethyleneoxy groups, and a peak at δ about 5.5 to about 6.5 ppm for 4 protons of acrylic groups.

### Example 3

### Synthesis of PTG65CD200

A methacrylate named PTG65CD200 of the formula below was synthesized by the method described below.

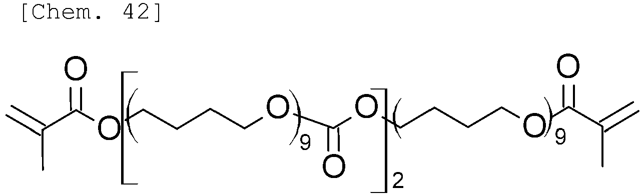

PTG65CD200 was synthesized using the same reaction process as in Example 1 except that NT2006 was used instead of NT1002.

The measured proton nuclear magnetic resonance spectrum of PTG65CD200 showed a peak at δ about 1.0 to about 2.5 ppm for about 114 protons of tetramethyleneoxy and methacrylic groups, a peak at δ about 3.0 to about 4.5 ppm for about 108 protons of tetramethyleneoxy groups, and a peak at δ about 5.5 to about 6.5 ppm for 4 protons of acrylic groups.

### Example 4

### Synthesis of UA-PTG25CD100

A urethane acrylate named UA-PTG25CD100 of the formula below was synthesized by the method described below.

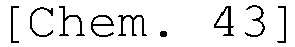

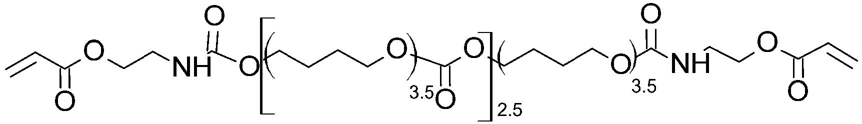

To 97.5 g of NT1002 was added 350 mL of dehydrated toluene and subjected to azeotropic dehydration. After 50 mL of toluene was removed by distillation, the mixture was cooled to 60°C, and 19.5 mg of p-methoxyphenol and 6.5 mg of dibutyltin dilaurate were added to the mixture. To the mixture was slowly added dropwise 27.2 g of 2-acryloyloxyethyl isocyanate. After the dropwise addition was completed, the mixture was reacted at 60 to 65°C for 8 hours. After the reaction was completed, 100 mL of water was added to the mixture and subjected to liquid-liquid separation. Celite was added to the resulting organic layer, stirred, and then filtered off. The resulting organic layer was concentrated to yield UA-PTG25CD100.

The measured proton nuclear magnetic resonance spectrum of UA-PTG25CD100 showed a peak at δ about 1.0 to about 2.5 ppm for about 49 protons of tetramethyleneoxy groups, a peak at δ about 3.0 to about 4.5 ppm for about 57 protons of tetramethyleneoxy and ethyleneoxy groups, and a peak at δ about 5.5 to about 6.5 ppm for 9 protons of acrylic groups.

### Production of Photochromic Curable Composition and Optical Component

### Components

Components (A)
Components of Formula (1A)
PTG25CD100: The methacrylate of Example 1
PTG25CD200: The methacrylate of Example 2
PTG65CD200: The methacrylate of Example 3
UA-PTG25CD100: The urethane acrylate of Example 4

### A-1

### M-PTMG65: Polytetramethylene glycol dimethacrylate (with an average molecular weight of 786)

### A-2

TMPT: Trimethylolpropane trimethacrylate
D-TMP: Ditrimethylolpropane tetramethacrylate
M-TMMT: Pentaerythritol tetramethacrylate
M-TMMT-80: A mixture of pentaerythritol trimethacrylate and pentaerythritol tetramethacrylate (18:82 in weight ratio)

### A-3

TSL: γ-Methacryloyloxypropyltrimethoxysilane
LA82: 1,2,2,6,6-Pentamethyl-4-piperidyl methacrylate

### A-4

14G: Polyethylene glycol dimethacrylate (with an average molecular weight of 770)
23G: Polyethylene glycol dimethacrylate (with an average molecular weight of 1,170)
MPCD100: Polycarbonate diol dimethacrylate (with an average molecular weight of 1,156) obtained by phosgenation of pentamethylene glycol and hexamethylene glycol

### A-5

RX-1: Acryloyl group-containing polyrotaxane having the following properties
Weight-average molecular weight Mw (GPC): 180,000
Acryloyl group modification ratio in the side chains: 80 mol% Percentage of OH groups remaining in the side chains: 20 mol% Axle molecule: Linear polyethylene glycol (PEG) with a molecular weight of 11,000
Threaded rings: α-Cyclodextrin (α-CD) with a degree of incorporation of 0.25
Axle molecule terminal: Blocked with adamantane
Side chains introduced into the threaded rings: Side chains with an (average) molecular weight of about 500
Number of acryloyl groups per molecule: About 90

RX-1 was synthesized using the method described in PCT International Publication No. WO2018/030275. The weight-average molecular weight Mw of RX-1 was measured using gel permeation chromatography (GPC). The measurement was performed using a liquid chromatography system (manufactured by Nihon Waters K.K.). The chromatography was performed using two columns TSKgel Super HM-M (molecular weight exclusion limit: 4,000,000, manufactured by Tosoh Corporation) in series. The eluent was tetrahydrofuran. The measurement was performed under the conditions of a flow rate of 0.6 mL/min and a temperature of 40°C. The standard sample was polystyrene, and the polystyrene-equivalent weight-average molecular weight was determined.

SO-1: Methacryloyl group-containing silsesquioxane having the following properties
Number of methacrylate groups per molecule: 20
Weight-average molecular weight: 4,800
SO-1 was synthesized using the method described below. First, 248 mL of ethanol and 54 g (3.0 mol) of water were added to 248 g (1.0 mol) of 3-trimethoxysilylpropyl methacrylate, and 0.20 g (0.005 mol) of sodium hydroxide was added as a catalyst to the mixture. The resulting mixture was reacted at 30°C for 3 hours. After the consumption of the starting material was confirmed by ¹H-NMR, the mixture was neutralized with dilute hydrochloric acid, and 174 mL of toluene, 174 mL of heptane, and 174 g of water were added to the mixture. The resulting aqueous layer was removed. Subsequently, the organic layer was washed with water until the aqueous layer became neutral. The organic layer was then concentrated by removal of the solvent to yield SO-1. SO-1 was determined by ²⁹Si-NMR to be a mixture of cage, ladder, and random structures.

The weight-average molecular weight Mw of SO-1 was measured using gel permeation chromatography (GPC). The measurement was performed using a liquid chromatography system (manufactured by Nihon Waters K.K.). The chromatography was performed using three columns Shodex GPC KF-802 (molecular weight exclusion limit: 5000, manufactured by Showa Denko K.K.), Shodex GPC KF-802.5 (molecular weight exclusion limit: 20,000, manufactured by Showa Denko K.K.), and Shodex GPC KF-803 (molecular weight exclusion limit: 70,000, manufactured by Showa Denko K.K.) in series. The eluent was tetrahydrofuran. The measurement was performed under the conditions of a flow rate of 1 mL/min and a temperature of 40°C. The standard sample was polystyrene, and the polystyrene-equivalent weight-average molecular weight was determined.

### Component (B)

PC1: A compound represented by the formula below.

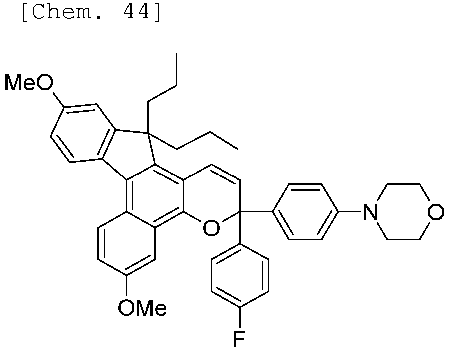

PC2: A compound represented by the formula below.

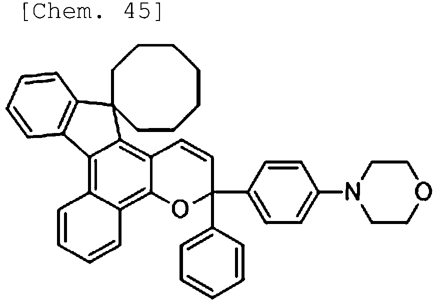

PC3: A compound represented by the formula below.

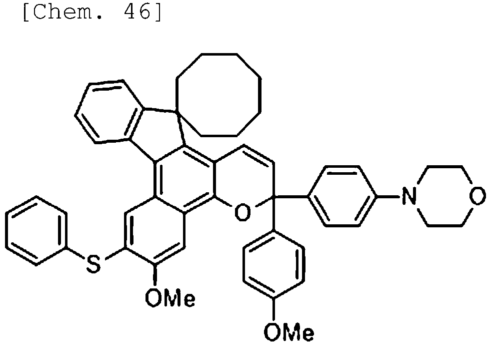

PC4: A compound represented by the formula below.

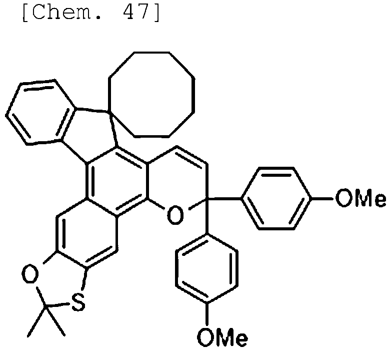

### Additional Additives

### Stabilizer

HALS: Bis(1,2,2,6,6-pentamethyl-4-piperidyl) sebacate HP: Ethylenebis(oxyethylene) bis[3-(5-tert-butyl-4-hydroxy-m-tolyl)propionate] (Irganox 245 manufactured by BASF Japan Co., Ltd.).

### Photopolymerization Initiator

### PI: Phenylbis(2,4,6-trimethylbenzoyl)-phosphine oxide (Omnirad 819 manufactured by IGM Resins M.V.)

### Example 5

Production of Photochromic Curable Composition First, the components were provided according to the formulation shown below.

### Components (A)

PTG25CD100 47.2 parts by mass
TMPT 47.2 parts by mass
TSL 5.6 parts by mass

### Component (B)

PC1 1.7 parts by mass

### Additional Additives

Polymerization initiator
PI 0.3 parts by mass
Stabilizers
HP 1 part by mass
HALS 3 parts by mass

Next, all compounds corresponding to the component (A) were mixed, and then the component (B) and the additional additives were added to form a mixture. To the resulting mixture was added 1,000 ppm of a leveling agent FZ-2110 manufactured by Dow Toray Co., Ltd. to form a photochromic curable composition.

### Production of Optical Component

A photochromic laminate was obtained by a lamination method using the photochromic curable composition.

First, a thiourethane-based plastic lens with a central thickness of 2 mm and a refractive index of 1.60 was provided as an optical base material. In advance, the thiourethane-based plastic lens was subjected to alkali etching with a 5% sodium hydroxide aqueous solution at 50°C for 5 minutes and then washed thoroughly with distilled water.

A moisture-curable primer (TR-SC-P (trade name) manufactured by Tokuyama Corporation) was applied to the surface of the plastic lens using a spin coater (1H-DX2 manufactured by Mikasa Co., Ltd.) at a rotation speed of 70 rpm for 15 seconds and then at a rotation speed of 700 rpm for 10 seconds. Subsequently, about 1 g of the photochromic curable composition obtained as described above was applied to the primer by spin coating to form a 40 µm-thick photochromic coating layer.

The lens with its surface coated with the photochromic curable composition (photochromic coating layer) was irradiated with light from a metal halide lamp at a power of 200 mW/cm² for 90 seconds in a nitrogen gas atmosphere so that the coating layer was cured. The lens was then heated at 90°C for 1 hour to give a photochromic laminate having a photochromic layer.

### Examples 6 to 18

Photochromic cured products were prepared as in Example 5, except that the photochromic curable compositions shown in Table 1 were used instead, and then evaluated for the same items as those for Example 5.

### Comparative Examples 1 to 4

Photochromic cured products were prepared as in Example 5, except that the photochromic curable compositions shown in Table 2 were used instead, and then evaluated for the same items as those for Example 5.

### Evaluation

The laminates obtained in the examples and comparative examples were evaluated according to the methodologies described below. The evaluation results are shown in Table 3.

### (1) Photochromic Properties

[1] Maximum Absorption Wavelength (λmax):
   Immediately after the laminate changed its color from colorless to colored, the maximum absorption wavelength of the laminate was determined using a spectrophotometer (instant multi-channel photodetector MCPD-3000 manufactured by Otsuka Electronics Co., Ltd.), and used as an indicator of color at the time of coloration.
[2] 23°C Color Optical Density (A₂₃):
   At 23°C, the difference between the absorbance ε(300) measured at the maximum absorption wavelength immediately after exposure to light for 300 seconds and the absorbance ε(0) measured before the exposure to light was determined and used as an indicator of color optical density. The higher the color optical density, the higher the photochromic performance is considered.
[3] 23°C Color Fading Half-Life (τ1/2 (sec)):
   At 23°C, the time required for the absorbance at the maximum absorption wavelength of the sample to decrease to half of {ε(300) - ε(0)} after the stopping of the 300-second exposure to light was determined and used as an indicator of color fading speed. The shorter the time, the faster the color fading speed.

### (2) Vickers Hardness

The Vickers hardness of the laminate was measured using a micro Vickers hardness tester PMT-X7A (manufactured by Matsuzawa Co., Ltd.). The measurement was performed using a square pyramid diamond indenter under the conditions of a load of 10 gf and an indenter holding time of 30 seconds. The measurement was performed four times in total. The first measurement with the largest measurement error was omitted, and the measurement result was defined as the average of the remaining three measurements.

### [Table 1]

**Table 1**

| Examples | Component A | | | | | | Component B | Additives |
|---|---|---|---|---|---|---|---|---|
| | Component of Formula (1A) | A-1 | A-2 | A-3 | A-4 | A-5 | | |
| Example 5 | PTG25CD100(47.2) | - | TMPT (47.2) | TSL(5.6) | - | - | PC1(1.7) | HALS (3) |
| | | | | | | | | HP(1) |
| | | | | | | | | PI (0.3) |
| Example 6 | PTG25CD200(47.2) | - | TMPT(47.2) | TSL(5.6) | - | - | PC1(1.7) | HALS (3) |
| | | | | | | | | HP(1) |
| | | | | | | | | PI (0.3) |
| Example 7 | PTG65CD200(47.2) | - | TMPT (47.2) | TSL(5.6) | - | - | PC1(1.7) | HALS (3) |
| | | | | | | | | HP(1) |
| | | | | | | | | PI (0.3) |
| Example 8 | PTG25CD100(37.8) | - | TMPT (25. 9) | TSL(5.6) | - | - | PC1(1.7) | HALS (3) |
| | | | | | | | | HP (1) |
| | PTG25CD200(30.7) | | | | | | | PI (0.3) |
| Example 9 | UA-PTG25CD100 (47.2) | - | TMPT (47.2) | TSL(5.6) | - | - | PC1(1.7) | HALS (3) |
| | | | | | | | | HP (1) |
| | | | | | | | | PI (0.3) |
| Example 10 | PTG25CD100(35.4) | - | TMPT(59.0) | TSL(5.6) | - | - | PC1(1.7) | HALS (3) |
| | | | | | | | | HP (1) |
| | | | | | | | | PI (0.3) |
| Example 11 | PTG25CD100(35.4) | M-PTMG65 (10.8) | TMPT(47.2) | TSL(5.6) | - | RX-1(1.0) | PC1(1.7) | HALS (3) |
| | | | | | | | | HP (1) |
| | | | | | | | | PI (0.3) |
| Example 12 | PTG25CD100(66.0) | - | D-TMP(25.5) | TSL(5.7) | - | SO-1(2.8) | PC1(1.7) | HALS (3) |
| | | | | | | | | HP (1) |
| | | | | | | | | PI (0.3) |
| Example 13 | PTG25CD100(32.4) | - | M-TMMT(13.9) | TSL(5.6) | - | - | PC1(1.7) | HP(1) |
| | PTG25CD200 (32.4) | | M-TMMT-80(13.9) | LA82(1.8) | | | | PI (0.3) |
| Example 14 | PTG25CD100(33.0) | - | TMPT (23. 6) | TSL(5.6) | 23G(23.6) | - | PC1(1.7) | HALS (3) |
| | | | | | | | | HP(1) |
| | | | M-TMMT (14.2) | | | | | PI (0.3) |
| Example 15 | PTG25CD200(44.0) | - | TMPT(18.8) | TSL(5.6) | 14G(12.6) | - | PC1(1.7) | HALS (3) |
| | | | M-TMMT(8.6) | | MPCD100 (10.4) | | | HP(1) |
| | | | | | | | | PI (0.3) |
| Example 16 | UA-PTG25CD100 (23.6) | - | TMPT(23.6) | TSL(5.5) | - | - | PC1(1.7) | HALS (3) |
| | | | M-TMMT-80(14.1) | | | | | HP(1) |
| | PTG65CD200(33.0) | | | | | | | PI (0.3) |
| Example 17 | PTG25CD100(12.3) | M-PTMG65 (12.3) | TMPT(16.5) | TSL(5.5) | 23G (12.3) | - | PC2(0.3) | HALS (3) |
| | PTG25CD200(12.3) | | M-TMMT-80 (16.5) | | | | | HP(1) |
| | PTG65CD200(12.3) | | | | | | PC4 (2.5) | PI (0.3) |
| Example 18 | PTG65CD200(33.0) | - | TMPT (20.8) | TSL(5.6) | 14G(23.6) | RX-1(2.8) | PC2(0.5) | HALS (3) |
| | | | | | | | PC3 (2.2) | HP(1) |
| | | | D-TMP (14.2) | | | | PC4(0.8) | PI (0.3) |

### [Table 2]

**Table 2**

| Comparative Examples | Component A | | | | | | Component B | Additives |
|---|---|---|---|---|---|---|---|---|
| | Component of Formula (1A) | A-1 | A-2 | A-3 | A-4 | A-5 | | |
| Comparative Example 1 | | | | | | | | HALS (3) |
| | - | - | TMPT(21.7) | TSL(5.6) | 14G(72.7) | - | PC1 (1.7) | HP(1) |
| | | | | | | | | PI (0.3) |
| Comparative Example 2 | | | | | | | | HALS (3) |
| | - | - | TMPT(47.2) | TSL(5.6) | 14G (47.2) | - | PC1 (1.7) | HP(1) |
| | | | | | | | | PI (0.3) |
| Comparative Example 3 | | | | | | | | HALS (3) |
| | - | - | TMPT(47.2) | TSL(5.6) | MPCD100 (47.2) | - | PC1 (1.7) | HP(1) |
| | | | | | | | | PI (0.3) |
| Comparative Example 4 | | | | | | | | HALS (3) |
| | - | - | TMPT(18.9) | TSL(5.6) | MPCD100 (75.5) | - | PC1 (1.7) | HP(1) |
| | | | | | | | | PI (0.3) |

### [Table 3]

**Table 3**

| | Photochromic properties | | | Vickers hardness |
|---|---|---|---|---|
| | Maximum absorption wavelength (nm) | 23°C color optical density (-) | 23°C color fading half-life (sec) | |
| Example 5 | 601 | 1.31 | 100 | 6.4 |
| Example 6 | 597 | 1.31 | 85 | 5.4 |
| Example 7 | 602 | 1.25 | 83 | 5.9 |
| Example 8 | 600 | 1.23 | 81 | 3.1 |
| Example 9 | 599 | 1.42 | 131 | 9.4 |
| Example 10 | 603 | 1.36 | 107 | 8.2 |
| Example 11 | 600 | 1.31 | 96 | 6.4 |
| Example 12 | 602 | 1.27 | 90 | 4.3 |
| Example 13 | 599 | 1.20 | 79 | 3.7 |
| Example 14 | 602 | 1.21 | 90 | 5.2 |
| Example 15 | 600 | 1.24 | 83 | 3.5 |
| Example 16 | 600 | 1.32 | 110 | 6.5 |
| Example 17 | 464 | 1.16 | 45 | 3.8 |
| | 569 | 0.83 | 45 | |
| Example 18 | 472 | 0.93 | 62 | 5.3 |
| | 583 | 1.00 | 62 | |
| Comparative Example 1 | 593 | 1.27 | 100 | 2.5 |
| Comparative Example 2 | 602 | 1.26 | 151 | 8.0 |
| Comparative Example 3 | 598 | 1.28 | 164 | 8.1 |
| Comparative Example 4 | 599 | 1.23 | 126 | 2.8 |

Preferred aspects of the present invention will be listed below.
[1] A (meth)acrylate represented by Formula (1A): 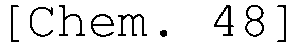 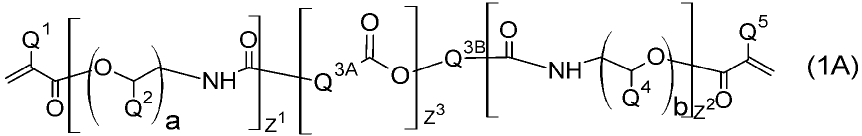 wherein
   Q¹ and Q⁵ are each independently a hydrogen atom or a methyl group,
   Q² and Q⁴ are each independently a hydrogen atom or an alkyl group having 1 to 3 carbon atoms,
   Q^{3A} and Q^{3B} are each independently a divalent group represented by Formula (1a) below,
   when two or more Q^{3A} are present, the two or more Q^{3A} may be the same group or different groups,
   a and b are each independently 0 or more and 10 or less,
   Z¹ and Z² are each independently 0 or 1,
   Z³ is 1 or more and 100 or less, 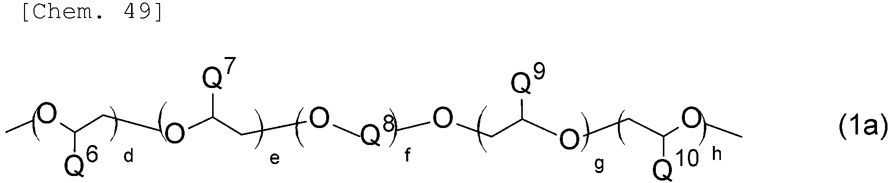 wherein
      Q⁶ and Q¹⁰ are each a hydrogen atom or an alkyl group having 1 to 3 carbon atoms,
      Q⁷ and Q⁹ are each a hydrogen atom or an alkyl group having 1 to 3 carbon atoms,
      Q⁶ and Q⁷ are different groups, and Q⁹ and Q¹⁰ are different groups,
      Q⁸ is an optionally substituted, linear or branched alkylene group having 2 to 20 carbon atoms,
      d and h are each 0 or more and 10 or less,
      e and g are each 0 or more and 20 or less, and f is 3 or more and 100 or less.
[2] The (meth)acrylate according to aspect [1], wherein d, e, g, and h are each 0.
[3] The (meth)acrylate according to aspect [1], wherein the (meth)acrylate is represented by Formula (1B): 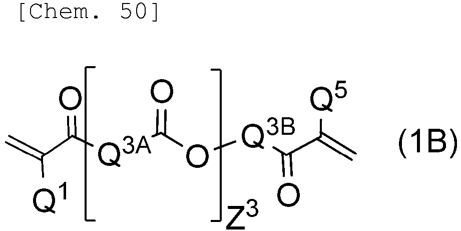 wherein
   Q¹, Q^{3A}, Q^{3B}, Q⁵, and Z³ are as defined for Formula (1A).
[4] The (meth)acrylate according to aspect [3], wherein Q⁸ in Formula (1a) is an optionally substituted, linear alkylene group having 3 to 9 carbon atoms.
[5] The (meth)acrylate according to aspect [1], wherein the (meth)acrylate is represented by Formula (1D): 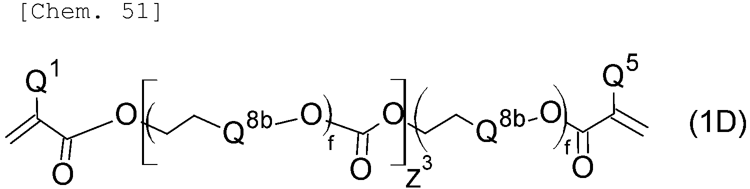 wherein
   Q¹, Q⁵, Z³, and f are as defined for Formula (1A), and
   Q^{8b} is a linear alkylene group having 1 to 7 carbon atoms.
[6] The (meth)acrylate according to aspect [1], wherein the (meth)acrylate has a number-average molecular weight of 100 or more and 10,000 or less.
[7] A curable composition comprising: the (meth)acrylate according to any one of aspects [1] to [6]; and a functional colorant.
[8] The curable composition according to aspect [7], wherein the curable composition has a functional colorant content of 0.001 mass% or more and 10 mass% or less.
[9] The curable composition according to aspect [7] or [8], wherein the curable composition has a (meth)acrylate content of 5 mass% or more and 99 mass% or less.
[10] The curable composition according to any one of aspects [7] to [9], further comprising a first radically polymerizable monomer represented by Formula (I): 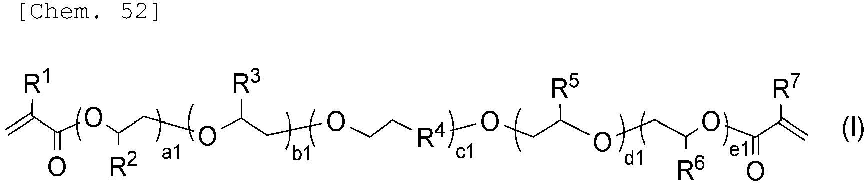 wherein
   R¹ and R⁷ are each independently a hydrogen atom or a methyl group,
   R² and R⁶ are each a hydrogen atom or an alkyl group having 1 to 3 carbon atoms,
   R³ and R⁵ are each a hydrogen atom or an alkyl group having 1 to 3 carbon atoms,
   R² and R³ are different groups, and R⁶ and R⁷ are different groups,
   R⁴ is an optionally substituted, linear or branched alkylene group having 1 to 7 carbon atoms,
   a1 and e1 are each 0 or more and 10 or less,
   b1 and d1 are each 0 or more and 20 or less,
   c1 is 2 or more and 100 or less, and greater than each of a1, b1, d1, and e1.
[11] The curable composition according to any one of aspects [7] to [10], further comprising a second radically polymerizable monomer having three or more (meth)acryloyl groups per molecule.
[12] The curable composition according to aspect [11], wherein the second radically polymerizable monomer comprises a multifunctional (meth)acrylate represented by Formula (2): 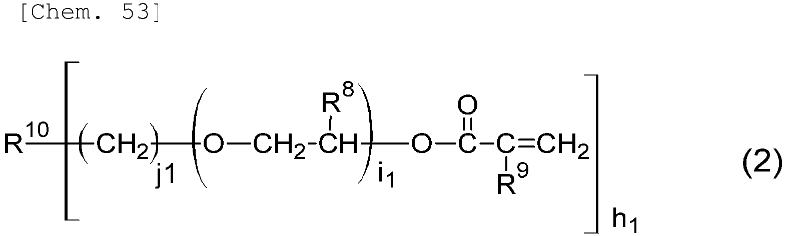 wherein
   R⁸ is a hydrogen atom or an alkyl group having 1 to 2 carbon atoms,
   R⁹ is a hydrogen atom or a methyl group,
   R¹⁰ is an organic group having a valence of 3 to 6 and having 1 to 10 carbon atoms,
   i1 is 0 or more and 3 or less,
   j1 is 0 or more and 3 or less, and
   h1 is 3 or more and 6 or less.
[13] The curable composition according to any one of aspects [7] to [12], further comprising a third radically polymerizable monomer having one (meth)acryloyl group per molecule.
[14] The curable composition according to any one of aspects [7] to [13], further comprising polyethylene glycol di(meth)acrylate.
[15] The curable composition according to any one of aspects [7] to [14], wherein the functional colorant comprises at least one compound selected from the group consisting of a chromene compound and a spirooxazine compound.
[16] A cured product comprising a product resulting from curing of the curable composition according to any one of aspects [7] to [15].
[17] A laminate comprising:
   an optical base material; and
   the cured product according to aspect [16] provided on a surface of the optical base material.
[18] An optical component comprising the cured product according to aspect [16].
[19] A lens comprising the cured product according to aspect [16].
[20] Eyeglasses comprising the lens according to aspect [19].

## Claims

1. A (meth)acrylate represented by Formula (lA): 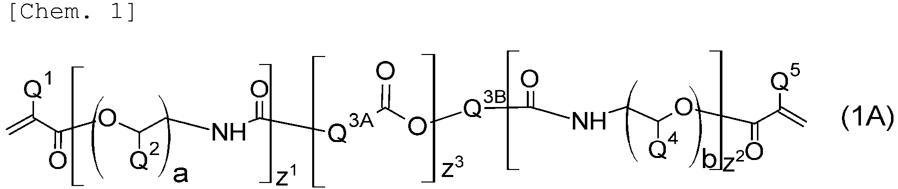 wherein
Q¹ and Q⁵ are each independently a hydrogen atom or a methyl group,
Q² and Q⁴ are each independently a hydrogen atom or an alkyl group having 1 to 3 carbon atoms,
Q^{3A} and Q^{3B} are each independently a divalent group represented by Formula (1a) below,
when two or more Q^{3A} are present, the two or more Q^{3A} may be the same group or different groups,
a and b are each independently 0 or more and 10 or less,
Z¹ and Z² are each independently 0 or 1,
Z³ is 1 or more and 100 or less, 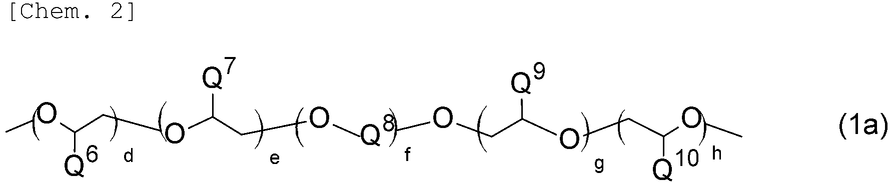
wherein
Q⁶ and Q¹⁰ are each a hydrogen atom or an alkyl group having 1 to 3 carbon atoms,
Q⁷ and Q⁹ are each a hydrogen atom or an alkyl group having 1 to 3 carbon atoms,
Q⁶ and Q⁷ are different groups, and Q⁹ and Q¹⁰ are different groups,
Q⁸ is an optionally substituted, linear or branched alkylene group having 2 to 20 carbon atoms,
d and h are each 0 or more and 10 or less,
e and g are each 0 or more and 20 or less, and
f is 3 or more and 100 or less.

2. The (meth)acrylate according to claim 1, wherein d, e, g, and h are each 0.

3. The (meth)acrylate according to claim 1, wherein the (meth)acrylate is represented by Formula (1B): 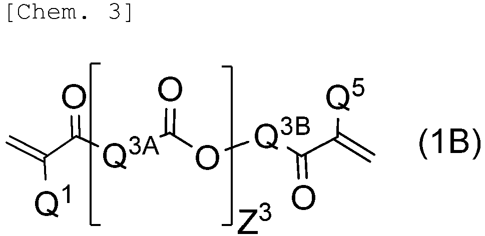 wherein
Q¹, Q^{3A}, Q^{3B}, Q⁵, and Z³ are as defined for Formula (1A).

4. The (meth)acrylate according to claim 3, wherein Q⁸ in Formula (1a) is an optionally substituted, linear alkylene group having 3 to 9 carbon atoms.

5. The (meth)acrylate according to claim 1, wherein the (meth)acrylate is represented by Formula (1D): 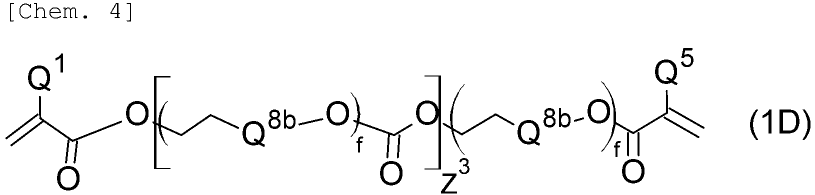 wherein
Q¹, Q⁵, Z³, and f are as defined for Formula (1A), and
Q^{8b} is a linear alkylene group having 1 to 7 carbon atoms.

6. The (meth)acrylate according to claim 1, wherein the (meth)acrylate has a number-average molecular weight of 100 or more and 10,000 or less.

7. A curable composition comprising: the (meth)acrylate according to claim 1; and a functional colorant.

8. The curable composition according to claim 7, wherein the curable composition has a functional colorant content of 0.001 mass% or more and 10 mass% or less.

9. The curable composition according to claim 7, wherein the curable composition has a (meth)acrylate content of 5 mass% or more and 99 mass% or less.

10. The curable composition according to claim 7, further comprising a first radically polymerizable monomer represented by Formula (I): 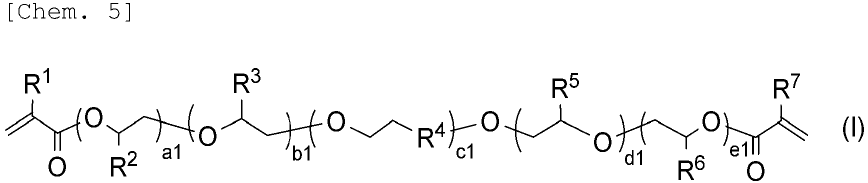 wherein
R¹ and R⁷ are each independently a hydrogen atom or a methyl group,
R² and R⁶ are each a hydrogen atom or an alkyl group having 1 to 3 carbon atoms,
R³ and R⁵ are each a hydrogen atom or an alkyl group having 1 to 3 carbon atoms,
R² and R³ are different groups, and R⁶ and R⁷ are different groups,
R⁴ is an optionally substituted, linear or branched alkylene group having 1 to 7 carbon atoms,
a1 and e1 are each 0 or more and 10 or less,
b1 and d1 are each 0 or more and 20 or less,
c1 is 2 or more and 100 or less, and greater than each of a1, b1, d1, and e1.

11. The curable composition according to claim 7, further comprising a second radically polymerizable monomer having three or more (meth)acryloyl groups per molecule.

12. The curable composition according to claim 11, wherein the second radically polymerizable monomer comprises a multifunctional (meth)acrylate represented by Formula (2): 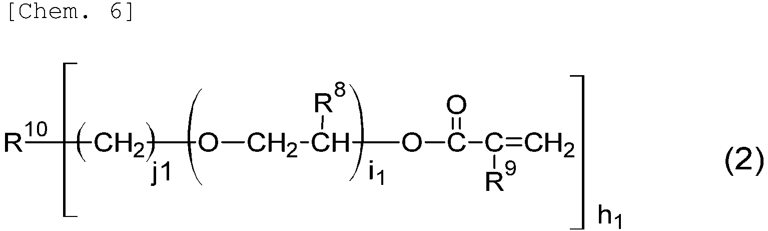 wherein
R⁸ is a hydrogen atom or an alkyl group having 1 to 2 carbon atoms,
R⁹ is a hydrogen atom or a methyl group,
R¹⁰ is an organic group having a valence of 3 to 6 and having 1 to 10 carbon atoms,
i1 is 0 or more and 3 or less,
j1 is 0 or more and 3 or less, and
h1 is 3 or more and 6 or less.

13. The curable composition according to claim 7, further comprising a third radically polymerizable monomer having one (meth)acryloyl group per molecule.

14. The curable composition according to claim 7, further comprising polyethylene glycol di(meth)acrylate.

15. The curable composition according to claim 7, wherein the functional colorant comprises at least one compound selected from the group consisting of a chromene compound and a spirooxazine compound.

16. A cured product comprising a product resulting from curing of the curable composition according to claim 7.

17. A laminate comprising:
an optical base material; and
the cured product according to claim 16 provided on a surface of the optical base material.

18. An optical component comprising the cured product according to claim 16.

19. A lens comprising the cured product according to claim 16.

20. Eyeglasses comprising the lens according to claim 19.
